# EUROPEAN PATENT APPLICATION

(11) **EP 4 421 166 A1**
(43) Date of publication of application: **28.08.2024**
(21) Application number: 23158085.3
(22) Date of filing: 22.02.2023
(51) Int. Cl.: C12N 9/14

(54) **IMPROVED SPLIT HALOTAGS**

(71) Applicant: Max-Planck-Gesellschaft zur Förderung der Wissenschaften e.V., 80539 München (DE)
(72) Inventor: WILHELM, Jonas, 69028 Heidelberg (DE); HINNAH, Konstantin, 69028 Heidelberg (DE); LIN, Yin-Hsi, 69028 Heidelberg (DE); NICKEL, Lennart, 69028 Heidelberg (DE); HIBLOT, Julien, 69028 Heidelberg (DE); JOHNSSON, Kai, 69028 Heidelberg (DE)
(74) Representative: Schulz Junghans Patentanwälte PartGmbB

(57) **Abstract**

The present invention relates to improved variants of the split-HaloTag system. HaloTag is a self-labelling protein tag derived from a bacterial protein and able to covalently bind to a synthetic ligand comprising a reactive chloroalkane. HaloTag can be split into a cpHaloΔ protein and a complementing peptide which are active only when brought into close proximity. The present invention relates to improved sequences of this split-HaloTag system that are more stable and show faster kinetics than the molecules of the art.

## Description

### Field

The present invention relates to improved variants of the split-HaloTag system. HaloTag is a self-labelling protein tag derived from a bacterial enzyme and able to covalently bind to synthetic ligands comprising a reactive chloroalkane. HaloTag can be split into a cpHaloΔ protein and a complementing peptide which are active only when brought into close proximity. The present invention relates to improved sequences of this split-HaloTag system being more stable and showing faster kinetics.

### Background

Methods for integrating biochemical processes over time are of great scientific interest. These biochemical processes encompass, for example, protein-protein interactions, changes in metabolite concentration or protein sub-cellular (re)localization. Currently, these biochemical processes are mainly studied employing real time fluorescence microscopy using tailor-made biosensors. Yet, fluorescence microscopy is restricted to confined fields of view and tissue depth. Despite great improvements in equipment, image processing and biosensor designs, it remains impossible to study fundamental events in complete rodent organs *in vivo* by fluorescence microscopy.

An alternative approach consists in the recording of biochemical processes that can be read out at a later time point. This recording process is defined as *integration.* It leads to an irreversible mark being accumulated over time in response to the biochemical process under investigation.

The *post hoc* evaluation of experiments is not only valuable for *in vivo* studies, but can also be beneficial for cell-based assays. Especially in the case of rarely occurring biochemical processes, the signal integration over time can improve the readout due to an enhanced signal to noise ratio. In high throughput screenings, signal integration could offer a snapshot of the studied phenomenon, thereby increasing multiplexing and reducing costs by replacing lengthy recordings via real-time microscopy. Finally, using fluorescent substrates, cell populations can be identified, and eventually sorted, based on their metabolic/signalling profile for downstream analysis and/or treatments.

Based on the self-labelling protein HaloTag, a chemogenetic integrator family of proteins was generated that has been engineered to irreversibly react with chloroalkane substrates in response to a given biochemical process. These integrators consist of split circular permutants of HaloTag that are complemented as a result of a change of conformation of a sensor in response to a biochemical process and invoke labelling activity as a result of this complementation. Circular permuted HaloTag is shown as SEQ ID NO 001. The original system is disclosed in WO2020212537, and US application no. 17/604,417. It comprises a first partial effector (SEQ ID NO 367; the cpHaloΔ protein) consisting of two components of the HaloTag protein (SEQ ID NO 002 and 003) joined by an internal linker, and complemented by a short second partial effector peptide (SEQ ID NO 004 or SEQ ID NO 005). However, there are several shortcomings of the original split-HaloTag system.

No complementation/labelling was observed when the split-HaloTag system was fused to termini of two interacting proteins that are rather distant (e.g., 47 Å). The K_{d} of the original Halo-Peptide (Hpep; SEQ ID NO 005) with the cpHaloΔ is 4.6 mM. Furthermore, the low thermostability of cpHaloΔ of 30 °C limits application in many cellular or *in vivo* systems.

Based on the above-mentioned state of the art, the objective of the present invention is to provide means and methods to design new Halo-Peptides with increased affinity to cpHaloΔ and to design of cpHaloΔ variants with increased thermal stability and high activity at 37 °C. This objective is attained by the subject-matter of the independent claims of the present specification, with further advantageous embodiments described in the dependent claims, examples, figures and general description of this specification.

### Summary of the Invention

A first aspect of the invention relates to a modular polypeptide complex comprising (or essentially consisting of) a first partial effector sequence constituting a cpHaloΔ protein, and a second partial effector sequence characterized in that the second partial effector sequence consists of a sequence selected from the group consisting of SEQ ID NO 6 to SEQ ID NO 343.

A second aspect of the invention relates to a modular polypeptide complex comprising (or essentially consisting of) a first partial effector sequence, and a second partial effector sequence characterized in that
a. the N-terminal first effector sequence part comprises at least one N-effector amino acid substitution; and/or
b. the C-terminal first effector sequence part comprises at least one C-effector amino acid substitution; and/or
c. the internal cpHalo linker is characterized by a novel sequence as provided herein, particularly a sequence selected from SEQ ID NO 344 to 366, listed below.

A third aspect of the invention relates to a modular polypeptide complex comprising the first partial effector sequence as described in the second aspect, and the second partial effector sequence as described in the first aspect.

Further aspects of the invention relate to a nucleic acid sequence or a nucleic acid expression system encoding the modular polypeptide complex, a cell or a non-human transgenic animal or plant comprising the modular polypeptide complex, a kit and a method for detection of a molecular interaction event.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *brought into close proximity* of *each other* in the context of the present specification relates to a situation where the first and second partial effector sequences are capable of interacting non-covalently. In certain embodiments, a sensor polypeptide is necessary to bring the first and second partial effector sequences close enough together to interact. In certain embodiments, the first and second partial effector sequences have a sufficiently high affinity towards each other that they can interact without the trigger of a sensor polypeptide. The first and second partial effector sequences in this case interact even if the first and second partial effector sequence are both just expressed inside the same cell or cell-free expression system.

The term *coupled to* in the context of the present specification relates to a covalent bond connecting the two partners which are coupled to each other. This covalent bond could be directly between the two partners or there could be another moiety in between.

The term *deletion* in the context of the present specification relates to an event that deletes one amino acid into a sequence, leading to a sequence having one amino acid less than the parent sequence.

The term *insertion* in the context of the present specification relates to an event that inserts one amino acid into a sequence, leading to a sequence having one amino acid more than the parent sequence.

The term *substitution* in the context of the present specification relates to an event that exchanges one "wild type" amino acid for another mutated or substituted amino acid at the same position in a sequence, leading to a sequence having one amino acid which differs from the amino acid at the same position inside the parent sequence (exchange in comparison to the parent sequence). A *variable substitution* relates to a substitution which does not decrease the function and activity of the parent sequence significantly. The variable substitution is a substitution retaining all the important features of the parent sequence. An *N-effector and the C-effector amino acid substitutions* are substitutions which improve a certain feature of the parent sequence as described in the specification. The N-effector and the C-effector amino acid substitutions are selected from a defined list of possible substitutions.

The term *detection moiety* in the context of the present specification relates to a chemical moiety which can be detected in solution, in a suspension of organic particles or inside a cell. The detection can be via, but is not limited to, light emission, radioactivity, or certain specific binding properties of the detection moiety. In certain embodiments, the detection moiety comprises a fluorophore. In certain particular embodiments, the detection moiety comprises a fluorescent dye molecule.

The term *purification tag* in the context of the present specification relates to a chemical moiety having a high affinity for and specific binding to an acceptor binding moiety. The binding of the purification tag to the acceptor moiety is specific and may be used to purify the moiety covalently coupled to the purification tag, e.g. via affinity chromatography. Non-limiting examples of a purification tag include T7-tag, Calmodulin-binding peptide, FLAG epitope, GST tag, HA tag, Polyhistidine tag, Myc tag, Biotin, Strep-tag.

The term *halogen alkane moiety* or *HaloTag substrate* in the context of the present specification relates to an ω-halogen alkyl moiety capable of covalent attachment to HaloTag proteins. In certain embodiments, a HaloTag substrate is a moiety of formula: 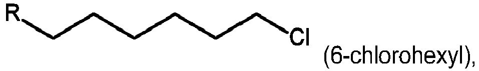 wherein R can be any moiety as further defined in the specification. In certain embodiments, R is a detection moiety. In certain particular embodiments, R comprises a fluorescent dye. In certain embodiments, R additionally comprises a linker. The HaloTag substrate can also be an ω-bromo-alkane, and aryl ω-halogen-alkyl substrates are known and can take this place (see Shields et al., Thousandfold Cell-Specific Pharmacology of Neurotransmission; BioRxiv Oct. 21, 2022).

In certain embodiments, non-covalent HaloTag substrates are encompassed by the term *halogen alkane moiety* or *HaloTag substrate.* Non-covalent HaloTag substrates are disclosed in WO 2022229466 A1, incorporated herein by reference.

The term "active" when used in relation to a HaloTag protein or variant thereof refers to the protein's ability to self-label, i.e. covalently attach to a HaloTag substrate, or -if the reference is made in context with a HaloTag system where non-covalent attachment to a HaloTag substrate as disclosed in WO 2022229466 is expected- non-covalent binding and release of the non-covalent HaloTag substrate.

The term *"hpep"* or *"hpeps"* (plural form) refers to the short, complementing peptide of the cp HaloTag system, also referred to as *second partial effector sequence* in describing the invention.

The term *in-frame insertion* of a *coding sequence* in the context of the present specification relates to the insertion of an open-reading frame at a position relative to another open-reading frame in a way that both open-reading frames can be transcribed and translated into one continuous peptide or polypeptide.

The term *multiple cloning site* in the context of the present specification relates to a short segment of DNA which contains several restriction sites, wherein each restriction site is uniquely cut by a specific restriction enzyme.

Any patent document cited herein shall be deemed incorporated by reference herein in its entirety.

### Sequences

Sequences similar or homologous to the sequences disclosed herein are also part of the invention (always provided that any mutation characterizing the invention is present and that the sequence is functional in the sense indicated herein). In some embodiments, the sequence identity at the amino acid level can be about 85%, 90%, 91%, 92%, 93%, 94%, 95%, 96%, 97%, 98%, 99% or higher. At the nucleic acid level, the sequence identity can be about 70%, 75%, 80%, 85%, 90%, 91%, 92%, 93%, 94%, 95%, 96%, 97%, 98%, 99% or higher. Alternatively, substantial identity exists when the nucleic acid segments will hybridize under selective hybridization conditions (e.g., very high stringency hybridization conditions), to the complement of the strand. The nucleic acids may be present in whole cells, in a cell lysate, or in a partially purified or substantially pure form.

In the context of the present specification, the terms *sequence identity* and *percentage* of *sequence identity* refer to a single quantitative parameter representing the result of a sequence comparison determined by comparing two aligned sequences position by position. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA. Software for performing BLAST analyses is publicly available, e.g., through the National Center for Biotechnology-Information (http://blast.ncbi.nlm.nih.gov/).

One example for comparison of amino acid sequences is the BLASTP algorithm that uses the default settings: Expect threshold: 10; Word size: 3; Max matches in a query range: 0; Matrix: BLOSUM62; Gap Costs: Existence 11, Extension 1; Compositional adjustments: Conditional compositional score matrix adjustment. One such example for comparison of nucleic acid sequences is the BLASTN algorithm that uses the default settings: Expect threshold: 10; Word size: 28; Max matches in a query range: 0; Match/Mismatch Scores: 1.-2; Gap costs: Linear. Unless stated otherwise, sequence identity values provided herein refer to the value obtained using the BLAST suite of programs (Altschul et al., J. Mol. Biol. 215:403-410 (1990)) using the above identified default parameters for protein and nucleic acid comparison, respectively.

Reference to identical sequences without specification of a percentage value implies 100% identical sequences (i.e. the same sequence).

### General Biochemistry: Peptides, Amino Acid Sequences

The term *polypeptide* in the context of the present specification relates to a molecule consisting of 50 or more amino acids that form a linear chain wherein the amino acids are connected by peptide bonds. The amino acid sequence of a polypeptide may represent the amino acid sequence of a whole (as found physiologically) protein or fragments thereof. The term "polypeptides" and "protein" are used interchangeably herein and include proteins and fragments thereof. Polypeptides are disclosed herein as amino acid residue sequences.

The term *peptide* in the context of the present specification relates to a molecule consisting of up to 50 amino acids, in particular 8 to 30 amino acids, more particularly 8 to 15amino acids, that form a linear chain wherein the amino acids are connected by peptide bonds.

Amino acid residue sequences are given from amino to carboxyl terminus. Capital letters for sequence positions refer to L-amino acids in the one-letter code (Stryer, Biochemistry, 3rd ed. p. 21). Lower case letters for amino acid sequence positions refer to the corresponding D- or (2R)-amino acids. Sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

The term *having substantially the same activity* in the context of the present specification relates to the activity of an effector polypeptide pair, i.e. haloalkane transferase activity. A polypeptide qualified as *having substantially the same activity* does not necessarily show the same quantity of activity as the reference polypeptide; in the particular case of the present invention, a reduction of (self-) labelling activity with respect to the reference peptide (SEQ ID NO 004 or 005) might indeed be desirable for certain applications. As laid out below, for purposes of distinguishing polypeptides covered by the present inventions from those that are not covered, the inventors propose a threshold of activity of 10² s⁻¹M⁻¹ in the standard assay as laid out in Example 3, with Halo-CPY as the substrate.

For purposes wherein the above definition of activity is not applicable, 3 standard deviations above background with regard to haloalkane transferase activity shall be taken as the reference threshold for *having substantially the same activity.* In certain embodiments, at least 5 standard deviations are used as the reference threshold. In certain particular embodiments, at least 10 standard deviations are used as the reference threshold.

In the context of the present specification, the term *amino acid linker* refers to a polypeptide of variable length that is used to connect two polypeptides in order to generate a single chain polypeptide. Unless specified otherwise, exemplary embodiments of linkers useful for practicing the invention specified herein are oligopeptide chains consisting of 1, 2, 3, 4, 5, 10, 20, 30, 40 or 50 amino acids.

### General Molecular Biology: Nucleic Acid Sequences, Expression

The term *nucleic acid expression vector* in the context of the present specification relates to a plasmid, a viral genome or an RNA, which is used to transfect (in case of a plasmid or an RNA) or transduce (in case of a viral genome) a target cell with a certain gene of interest, or -in the case of an RNA construct being transfected- to translate the corresponding protein of interest from a transfected mRNA. For vectors operating on the level of transcription and subsequent translation, the gene of interest is under control of a promoter sequence and the promoter sequence is operational inside the target cell, thus, the gene of interest is transcribed either constitutively or in response to a stimulus or dependent on the cell's status. In certain embodiments, the viral genome is packaged into a capsid to become a viral vector, which is able to transduce the target cell.

### Markers, ligands

The term *fluorescent dye* or *fluorophore* in the context of the present specification relates to a small molecule capable of fluorescence in the visible or near infrared spectrum. Examples for fluorescent labels or labels presenting a visible color include, without being restricted to, fluorescein, rhodamine and silcon-rhodamine based dyes, allophycocyanine (APC), peridinin chlorophyll (PerCP), phycoerithrin (PE), Alexa Fluors (Life Technologies, Carlsbad, CA, USA), DyLight fluors (Thermo Fisher Scientific, Waltham, MA, USA)ATTO Dyes (ATTO-TEC GmbH, Siegen, Germany), BODIPY Dyes (4,4-difluoro-4-bora-3a,4a-diaza-s-indacene based dyes) and the like. The term *fluorescent dye or fluorophore* in the context of the present specification also relates to dyes described in WO2020115286 and WO2019122269A1, or US16956596, which are incorporated by reference herein.

### Detailed Description of the Invention

### Improved labelling speed:

A first aspect of the invention relates to a modular polypeptide complex comprising (or essentially consisting of) a first partial effector sequence, and a second partial effector sequence characterized in that the second partial effector sequence consists of a sequence selected from the group consisting of SEQ ID NO 6 to SEQ ID NO 343.

The improved sequences provided by the invention enable tuning of the affinity between the two distinct, split parts of the HaloTag protein, which allows much broader usage of the tool in view of the many distinct peptides it may be connected to (sensors, see below). The peptides provided by the invention cover a broad range of affinities, enabling a range of different applications of the technology.

Optionally, the second partial effector sequence may comprise one or two amino acid variations independently selected from a deletion, an insertion and a variable substitution.

The first and second partial effector sequences together constitute an active, circularly permuted, haloalkane dehalogenase derived, self-labeling protein, and are capable, when brought into close proximity of each other, to effect covalent attachment of a halogen alkane moiety to the first partial effector sequence.

The first partial effector sequence consists of
∘ an N-terminal first effector sequence part characterized by SEQ ID NO 002 (the original N-terminal cpHaloΔ protein) or by a sequence at least (≥) 90% identical (particularly ≥93%, 95%, 97% or ≥98% identical) to SEQ ID NO 002,
∘ a C-terminal first effector sequence part characterized by SEQ ID NO 003 (the original C-terminal cpHaloΔ protein) or by a sequence at least (≥) 90% identical (particularly ≥93%, 95%, 97% or ≥98% identical) to SEQ ID NO 003,
   an internal cpHalo linker consisting of 10 to 35 amino acids, wherein the internal cpHalo linker connects the C-terminus of the N-terminal first effector sequence part to the N-terminus of the C-terminal first effector sequence part.

In particular embodiments, the C-terminal effector sequence part may be characterized by a variant of SEQ ID NO 003 wherein the D (aspartic acid) residue in the active centrum of the protein has been exchanged, for example to A (see SEQ ID NO 370), to give rise to a class of non-covalent HaloTag mutants that bind substrates, but do not covalently attach them, which offers applications particularly in advanced (super-resolution) microscopy techniques. The inventors have been able to ascertain that these non-covalent HaloTag systems profit from the invention as herein described in any of its aspects.

In certain embodiments, the internal cpHalo linker consists of 12 to 20 amino acids. In certain embodiments, the internal cpHalo linker consists of -15 amino acids.

An alternative of this first aspect of the invention provides the isolated second partial effector sequence described by any of SEQ ID NO 6 to SEQ ID NO 343, which can be combined with the first partial effector sequence alone as a kit, or in combination, either as part of two separate fusion proteins comprising the first and second partial effector sequences, respectively, or as part of a larger fusion protein.

In certain embodiments, the second partial effector sequence consists of a sequence selected from the group consisting of SEQ ID NO: 6-10, 12,13, 15-26, 28, 30, 31, 34, 40, 46, 48, 50, 75, 85, 98, 103, 112, 154, 156, 160, 167, 173, 175, 182, 189, 195, 212, 214, 233 and 337-342, wherein optionally, the second partial effector sequence may comprise one or two amino acid variations independently selected from a deletion, an insertion and a variable substitution.

In certain embodiments, the second partial effector sequence consists of a sequence selected from the group consisting of
- WREEVRKAFKLFRQ (SEQ ID NO: 25)
- WREEVRKAFKLFRS (SEQ ID NO: 24)
- WRETFQLFRT (SEQ ID NO: 26)
- WREMFRLFRTGRVQ (SEQ ID NO: 27)
- WREMFQAFRT (SEQ ID NO: 28)
- WREMFRLFRTGQRS (SEQ ID NO: 29)
- WREMFQLFRT (SEQ ID NO: 30)
- WREMFRLFRT (SEQ ID NO: 20)
- SKRDWREMFRLFRT (SEQ ID NO: 31)
- RVMSWREMFRLFRT (SEQ ID NO: 32)
- RMWSWREMFRLFRT (SEQ ID NO: 33)
- WKRDWREMFRLFRT (SEQ ID NO: 34)
- RQWTWREMFRLFRT (SEQ ID NO: 35)
- RGWTWREMFRLFRT (SEQ ID NO: 36)
- RMWTWREMFRLFRT (SEQ ID NO: 37)
- RQWSWREMFRLFRT (SEQ ID NO: 38)
- RGWSWREMFRLFRT (SEQ ID NO: 39)
wherein optionally, the second partial effector sequence may comprise one or two amino acid variations independently selected from a deletion, an insertion and a variable substitution.

In certain embodiments, the second partial effector sequence consists of WREEVRKAFKLFRQ (SEQ ID NO: 25). In certain embodiments, the second partial effector sequence consists of WREEVRKAFKLFRS (SEQ ID NO: 24). In certain embodiments, the second partial effector sequence consists of WRETFQLFRT (SEQ ID NO: 26). In certain embodiments, the second partial effector sequence consists of WREMFRLFRTGRVQ (SEQ ID NO: 27). In certain embodiments, the second partial effector sequence consists of WREMFQAFRT (SEQ ID NO: 28). In certain embodiments, the second partial effector sequence consists of WREMFRLFRTGQRS (SEQ ID NO: 29). In certain embodiments, the second partial effector sequence consists of WREMFQLFRT (SEQ ID NO: 30). In certain embodiments, the second partial effector sequence consists of WREMFRLFRT (SEQ ID NO: 20). In certain embodiments, the second partial effector sequence consists of SKRDWREMFRLFRT (SEQ ID NO: 31). In certain embodiments, the second partial effector sequence consists of RVMSWREMFRLFRT (SEQ ID NO: 32). In certain embodiments, the second partial effector sequence consists of RMWSWREMFRLFRT (SEQ ID NO: 33). In certain embodiments, the second partial effector sequence consists of WKRDWREMFRLFRT (SEQ ID NO: 34). In certain embodiments, the second partial effector sequence consists of RQWTWREMFRLFRT (SEQ ID NO: 35). In certain embodiments, the second partial effector sequence consists of RGWTWREMFRLFRT (SEQ ID NO: 36). In certain embodiments, the second partial effector sequence consists of RMWTWREMFRLFRT (SEQ ID NO: 37). In certain embodiments, the second partial effector sequence consists of RQWSWREMFRLFRT (SEQ ID NO: 38). In certain embodiments, the second partial effector sequence consists of RGWSWREMFRLFRT (SEQ ID NO: 39).

In certain embodiments, the second partial effector sequence consists of a sequence selected from the group consisting of
- SKRDAREMFQAFRT (SEQ ID NO: 6);
- ARLFFQLFRT (SEQ ID NO: 7);
- YFQGARETFQAFRT (SEQ ID NO: 8);
- WIETFKLYRE (SEQ ID NO: 9);
- RKKEARETFQAFRT (SEQ ID NO: 10);
- VIETFKLFRS (SEQ ID NO: 11);
- AREMFQLFRT (SEQ ID NO: 12);
- AYKIFQLFRT (SEQ ID NO: 13);
- AIRMFQLFRT (SEQ ID NO: 14);
- WGDEARETFQAFRT (SEQ ID NO: 15);
- AREMFQAFRT (SEQ ID NO: 16);
- WKDEVIDAFRKFRE (SEQ ID NO: 17);
- ERETWQAFRT (SEQ ID NO: 18);
- WREEVRKTFKLFRQ (SEQ ID NO: 19);
- WREMFRLFRT (SEQ ID NO: 20);
- YFQGAREMFQAFRT (SEQ ID NO: 21);
- WKEEVIKAFKLFRD (SEQ ID NO: 22);
- AVNMFQLFRT (SEQ ID NO: 23);
- WREEVRKAFKLFRS (SEQ ID NO: 24);
wherein optionally, the second partial effector sequence may comprise one or two amino acid variations independently selected from a deletion, an insertion and a variable substitution.

In certain embodiments, the second partial effector sequence consists of SKRDAREMFQAFRT (SEQ ID NO: 6). In certain embodiments, the second partial effector sequence consists of ARLFFQLFRT (SEQ ID NO: 7). In certain embodiments, the second partial effector sequence consists of YFQGARETFQAFRT (SEQ ID NO: 8). In certain embodiments, the second partial effector sequence consists of WIETFKLYRE (SEQ ID NO: 9). In certain embodiments, the second partial effector sequence consists of RKKEARETFQAFRT (SEQ ID NO: 10). In certain embodiments, the second partial effector sequence consists of VIETFKLFRS (SEQ ID NO: 11). In certain embodiments, the second partial effector sequence consists of AREMFQLFRT (SEQ ID NO: 12). In certain embodiments, the second partial effector sequence consists of AYKIFQLFRT (SEQ ID NO: 13). In certain embodiments, the second partial effector sequence consists of AIRMFQLFRT (SEQ ID NO: 14). In certain embodiments, the second partial effector sequence consists of WGDEARETFQAFRT (SEQ ID NO: 15). In certain embodiments, the second partial effector sequence consists of AREMFQAFRT (SEQ ID NO: 16). In certain embodiments, the second partial effector sequence consists of WKDEVIDAFRKFRE (SEQ ID NO: 17). In certain embodiments, the second partial effector sequence consists of ERETWQAFRT (SEQ ID NO: 18). In certain embodiments, the second partial effector sequence consists of WREEVRKTFKLFRQ (SEQ ID NO: 19). In certain embodiments, the second partial effector sequence consists of WREMFRLFRT (SEQ ID NO: 20). In certain embodiments, the second partial effector sequence consists of YFQGAREMFQAFRT (SEQ ID NO: 21). In certain embodiments, the second partial effector sequence consists of WKEEVIKAFKLFRD (SEQ ID NO: 22). In certain embodiments, the second partial effector sequence consists of AVNMFQLFRT (SEQ ID NO: 23). In certain embodiments, the second partial effector sequence consists of WREEVRKAFKLFRS (SEQ ID NO: 24).

The first partial effector sequences as given in SEQ ID NO 367 and 371 may be used together with the improved second partial effector peptides claimed as this first aspect of the invention, as may be any cpHaloΔ variants claimed in the second aspect of the invention described below:

### Improved cpHaloΔ:

A second aspect of the invention relates to a modular polypeptide complex comprising (or essentially consisting of) a first partial effector sequence, and a second partial effector sequence characterized in that
a. the N-terminal first effector sequence part comprises at least one N-effector amino acid substitution; and/or
b. the C-terminal first effector sequence part comprises at least one C-effector amino acid substitution; and/or
c. the internal cpHalo linker is characterized by a sequence selected from SEQ ID NO 344 to 366.

The first and second partial effector sequences together constitute an active, circularly permuted, self-labeling protein, and are capable, when brought into close proximity of each other, to effect covalent attachment of a halogen alkane moiety to the first partial effector sequence.

The second partial effector sequence consists of a sequence selected from
- SEQ ID NO 004 and SEQ ID NO 005 (original cpHalo peptides), or
- any one of SEQ ID NO 6 to SEQ ID NO 343, or a subset thereof as laid out according to the first aspect to the invention herein;
wherein optionally, the second partial effector sequence may comprise one or two amino acid variations independently selected from a deletion, an insertion and a variable substitution.

The first partial effector sequence is a variant of SEQ ID NO 367 and 371, in other words differs from these known sequences, and consists of
∘ an N-terminal first effector sequence part characterized by SEQ ID NO 002 or by a sequence at least (≥) 90% identical (particularly ≥93%, 95%, 97% or ≥98% identical) to SEQ ID NO 002;
∘ a C-terminal first effector sequence part characterized by SEQ ID NO 003 or by a sequence at least (≥) 90% identical (particularly ≥93%, 95%, 97% or ≥98% identical) to SEQ ID NO 003, or a noncovalent variant of SEQ ID NO 003, such as embodied by SEQ ID NO 370;
∘ an internal cpHalo linker consisting of 10 to 35 amino acids, wherein the internal cpHalo linker connects the C-terminus of the N-terminal first effector sequence part to the N-terminus of the C-terminal first effector sequence part.

In certain embodiments, the internal cpHalo linker consists of 20 to 26 amino acids. In certain embodiments, the internal cpHalo linker consists of 22 to 24 amino acids.

The first partial effector sequences as given in SEQ ID NO 367 and 371 are not claimed as part of this second aspect of the invention, which relates to improved cpHaloΔ variants.

In certain embodiments, only the N-terminal first effector sequence part comprises at least one N-effector amino acid substitution. In certain embodiments, only the C-terminal first effector sequence part comprises at least one C-effector amino acid substitution. In certain embodiments, both N- and C-terminal first effector sequences are as published, and only the internal cpHalo linker is different, characterized by a sequence as newly disclosed herein and listed below.

In certain embodiments, the N-terminal first effector sequence part comprises at least one N-effector amino acid substitution and the C-terminal first effector sequence part comprises at least one C-effector amino acid substitution. In certain embodiments, the N-terminal first effector sequence part comprises at least one N-effector amino acid substitution and the internal cpHalo linker is characterized by a certain sequence listed below. In certain embodiments, the C-terminal first effector sequence part comprises at least one C-effector amino acid substitution and the internal cpHalo linker is characterized by a certain sequence listed below.

In certain embodiments, the N-terminal first effector sequence part comprises at least one N-effector amino acid substitution and the C-terminal first effector sequence part comprises at least one C-effector amino acid substitution and the internal cpHalo linker is characterized by a certain sequence listed below.

The N-effector amino acid substitution (mutation) is selected from the group consisting of
∘ N217D;
∘ F205W;
∘ V184E;
∘ V197K;
∘ R254W.

In certain embodiments, the N-effector amino acid substitution is N217D. In certain embodiments, the N-effector amino acid substitution is F205W. In certain embodiments, the N-effector amino acid substitution is V184E. In certain embodiments, the N-effector amino acid substitution is V197K. In certain embodiments, the N-effector amino acid substitution is R254W.

The C-effector amino acid substitution (mutation) is selected from the group consisting of
∘ F80T;
∘ N119H;
∘ K117R;
∘ R30P;
∘ G7D;
∘ E20S.

In certain embodiments, the C-effector amino acid substitution is F80T. In certain embodiments, the C-effector amino acid substitution is N119H. In certain embodiments, the C-effector amino acid substitution is K117R. In certain embodiments, the C-effector amino acid substitution is R30P. In certain embodiments, the C-effector amino acid substitution is G7D. In certain embodiments, the C-effector amino acid substitution is E20S.

The numbering of the N-effector and C-effector amino acid substitutions refers to the sequence position numbering of original halo7 sequence of GenBank-ID: AQS79242.1 (original halo7 sequence without permutation).

In certain embodiments, the internal cpHalo linker is characterized by a sequence selected from the group consisting of
∘ RSDDPRKTQTIASKISRDLNGS (SEQ ID NO: 344);
∘ KGGTKRDADKAVRDTLLSLNGQ (SEQ ID NO: 345);
∘ GGAPRDEALKKIEKAKRDTGDQ (SEQ ID NO: 346);
∘ KSKYDRDQILKIIAELEKKTGGS (SEQ ID NO: 347);
∘ QSKYPPEWLEKVIRELLKRKNGR (SEQ ID NO: 348);
∘ KSKYDKRQIRDIADKIAKDNNHQ (SEQ ID NO: 349);
∘ GADDKTKIEKILEEIKRRWQGR (SEQ ID NO: 350);
∘ GTSDPRNQEIAKKLARDASTVP (SEQ ID NO: 351);
∘ NGADKEQIDRAIEKAKRDLNNQ (SEQ ID NO: 352);
∘ KGASDRDEAKKLADDIRKKKGDQ (SEQ ID NO: 353);
∘ NSNGHRDELEKILQTIRKQNNDI (SEQ ID NO: 354);
∘ LKDERQRDKALEIADRADKYPTS (SEQ ID NO: 355);
∘ KGAEDAKERLERGDIEKKKKEQP (SEQ ID NO: 356);
∘ KGAEDAKERLERGDLDRWSQEWR (SEQ ID NO: 357);
∘ KGAEDAKERLERRDLDKINSRNS (SEQ ID NO: 358);
∘ KGAEDAKERLEKGYIDDKASKQQ (SEQ ID NO: 359);
∘ KGAEDAKERLEKGELEKKWKDHP (SEQ ID NO: 360);
∘ KGAEDAKERLERGEMEKAVKHGS (SEQ ID NO: 361);
∘ KGAEDAKERLERDELTRDIKTYPY (SEQ ID NO: 362);
∘ KGAEDAKERLEQGMLEEIKKKYPE (SEQ ID NO: 363);
∘ KGAEDAKERLERDELTKIAKNLGG (SEQ ID NO: 364);
∘ KGAEDAKERLEKNALDKIAKSKGD (SEQ ID NO: 365);
∘ KGAEDAKERLEKNDETLKKAKDKP (SEQ ID NO: 366);
wherein optionally, the internal cpHalo linker sequence may comprise one or two or three amino acid variations independently selected from a deletion, an insertion and a variable substitution.

In certain embodiments, the internal cpHalo linker is characterized by a sequence RSDDPRKTQTIASKISRDLNGS (SEQ ID NO: 344). In certain embodiments, the internal cpHalo linker is characterized by a sequence KGGTKRDADKAVRDTLLSLNGQ (SEQ ID NO: 345). In certain embodiments, the internal cpHalo linker is characterized by a sequence GGAPRDEALKKIEKAKRDTGDQ (SEQ ID NO: 346). In certain embodiments, the internal cpHalo linker is characterized by a sequence KSKYDRDQILKIIAELEKKTGGS (SEQ ID NO: 347). In certain embodiments, the internal cpHalo linker is characterized by a sequence QSKYPPEWLEKVIRELLKRKNGR (SEQ ID NO: 348). In certain embodiments, the internal cpHalo linker is characterized by a sequence KSKYDKRQIRDIADKIAKDNNHQ (SEQ ID NO: 349). In certain embodiments, the internal cpHalo linker is characterized by a sequence GADDKTKIEKILEEIKRRWQGR (SEQ ID NO: 350). In certain embodiments, the internal cpHalo linker is characterized by a sequence GTSDPRNQEIAKKLARDASTVP (SEQ ID NO: 351). In certain embodiments, the internal cpHalo linker is characterized by a sequence NGADKEQIDRAIEKAKRDLNNQ (SEQ ID NO: 352). In certain embodiments, the internal cpHalo linker is characterized by a sequence KGASDRDEAKKLADDIRKKKGDQ (SEQ ID NO: 353). In certain embodiments, the internal cpHalo linker is characterized by a sequence NSNGHRDELEKILQTIRKQNNDI (SEQ ID NO: 354). In certain embodiments, the internal cpHalo linker is characterized by a sequence LKDERQRDKALEIADRADKYPTS (SEQ ID NO: 355). In certain embodiments, the internal cpHalo linker is characterized by a sequence KGAEDAKERLERGDIEKKKKEQP (SEQ ID NO: 356). In certain embodiments, the internal cpHalo linker is characterized by a sequence KGAEDAKERLERGDLDRWSQEWR (SEQ ID NO: 357). In certain embodiments, the internal cpHalo linker is characterized by a sequence KGAEDAKERLERRDLDKINSRNS (SEQ ID NO: 358). In certain embodiments, the internal cpHalo linker is characterized by a sequence KGAEDAKERLEKGYIDDKASKQQ (SEQ ID NO: 359). In certain embodiments, the internal cpHalo linker is characterized by a sequence KGAEDAKERLEKGELEKKWKDHP (SEQ ID NO: 360). In certain embodiments, the internal cpHalo linker is characterized by a sequence KGAEDAKERLERGEMEKAVKHGS (SEQ ID NO: 361). In certain embodiments, the internal cpHalo linker is characterized by a sequence KGAEDAKERLERDELTRDIKTYPY (SEQ ID NO: 362). In certain embodiments, the internal cpHalo linker is characterized by a sequence KGAEDAKERLEQGMLEEIKKKYPE (SEQ ID NO: 363). In certain embodiments, the internal cpHalo linker is characterized by a sequence KGAEDAKERLERDELTKIAKNLGG (SEQ ID NO: 364). In certain embodiments, the internal cpHalo linker is characterized by a sequence KGAEDAKERLEKNALDKIAKSKGD (SEQ ID NO: 365). In certain embodiments, the internal cpHalo linker is characterized by a sequence KGAEDAKERLEKNDETLKKAKDKP (SEQ ID NO: 366).

In certain embodiments, the first partial effector sequence comprises N-effector amino acid substitutions and/or C-effector amino acid substitutions and/or internal cpHalo linker sequences selected from the group consisting of:
- E20S and V184E;
- E20S, N119H, and V184E;
- E20S, N119H, V184E, and V197K;
- E20S, N119H, and V184E, and linker sequence KSKYDRDQILKIIAELEKKTGGS (SEQ ID NO: 347);
- E20S, N119H, V184E, and V197K, and linker sequence KSKYDRDQILKIIAELEKKTGGS (SEQ ID NO: 347).

It is understood that the variants of the previously disclosed HaloTag systems as provided herein, also encompass any analogous variant of the non-covalent HaloTag system, wherein the nucleophilic D residue has been replaced, for example by A (see SEQ ID NO 371).

In certain embodiments, the first partial effector sequence comprises E20S, N119H, V184E, and V197K, and linker sequence KSKYDRDQILKIIAELEKKTGGS (SEQ ID NO: 347).

### Combination of improved-labelling-speed peptides and improved cpHaloΔ:

A third aspect of the invention relates to a modular polypeptide complex comprising the first partial effector sequence as described in the second aspect, and the second partial effector sequence as described in the first aspect.

The second partial effector sequence consists of a sequence selected from
- SEQ ID NO 004 and SEQ ID NO 005 (original cpHalo peptides), or
- any one of SEQ ID NO 6 to SEQ ID NO 343, or a subset thereof as laid out according to the first aspect to the invention herein.

In certain embodiments, the second partial effector sequence consists of a sequence selected from the group consisting of any one of SEQ ID NO 6 to SEQ ID NO 343, and the N-terminal first effector sequence part comprises at least one N-effector amino acid substitution selected from the group of N217D, F205W, V184E, V197K, R254W.

In certain embodiments, the second partial effector sequence consists of a sequence selected from the group consisting of any one of SEQ ID NO 6 to SEQ ID NO 343, and the C-terminal first effector sequence part comprises at least one C-effector amino acid substitution selected from the group of F80T, N119H, K117R, R30P, G7D, E20S.

In certain embodiments, the second partial effector sequence consists of a sequence selected from the group consisting of any one of SEQ ID NO 6 to SEQ ID NO 343, and the internal cpHalo linker is characterized by a sequence selected from the group consisting of SEQ ID NO 344 to SEQ ID NO 366.

In certain embodiments, the second partial effector sequence consists of a sequence selected from the group consisting of any one of SEQ ID NO 6 to SEQ ID NO 343, and
- the N-terminal first effector sequence part comprises at least one N-effector amino acid substitution selected from the group of N217D, F205W, V184E, V197K, R254W; and
- the C-terminal first effector sequence part comprises at least one C-effector amino acid substitution selected from the group of F80T, N119H, K117R, R30P, G7D, E20S.

In certain embodiments, the second partial effector sequence consists of a sequence selected from the group consisting of any one of SEQ ID NO 6 to SEQ ID NO 343, and
- the N-terminal first effector sequence part comprises at least one N-effector amino acid substitution selected from the group of N217D, F205W, V184E, V197K, R254W; and
- the internal cpHalo linker is characterized by a sequence selected from the group consisting of SEQ ID NO 344 to SEQ ID NO 366.

In certain embodiments, the second partial effector sequence consists of a sequence selected from the group consisting of any one of SEQ ID NO 6 to SEQ ID NO 343, and
- the C-terminal first effector sequence part comprises at least one C-effector amino acid substitution selected from the group of F80T, N119H, K117R, R30P, G7D, E20S, and
- the internal cpHalo linker is characterized by a sequence selected from the group consisting of SEQ ID NO 344 to SEQ ID NO 366.

In certain embodiments, the second partial effector sequence consists of a sequence selected from the group consisting of any one of SEQ ID NO 6 to SEQ ID NO 343, and
- the N-terminal first effector sequence part comprises at least one N-effector amino acid substitution selected from the group of N217D, F205W, V184E, V197K, R254W; and
- the C-terminal first effector sequence part comprises at least one C-effector amino acid substitution selected from the group of F80T, N119H, K117R, R30P, G7D, E20S, and
- the internal cpHalo linker is characterized by a sequence selected from the group consisting of SEQ ID NO 344 to SEQ ID NO 366.

In certain embodiments, the second partial effector sequence consists of a sequence selected from the group consisting of any one of SEQ ID NO 006-010, 012, 013, 015-026, 028, 030, 031, 034, 040, 046, 048, 050, 075, 085, 098, 103, 112, 154, 156, 160, 167, 173, 175, 182, 189, 195, 212, 214, 233 and 337-342, and the first partial effector sequence comprises at least one feature selected from an N-effector amino acid substitution, a C-effector amino acid substitution, a novel internal cpHalo linker as provided herein (particularly a sequence selected from SEQ ID NO 344 to 366).

In certain embodiments, the second partial effector sequence consists of a sequence selected from the group consisting of any one of SEQ ID NO 020 or 024-039, and the first partial effector sequence comprises at least one feature selected from an N-effector amino acid substitution, a C-effector amino acid substitution, a novel internal cpHalo linker as provided herein (particularly a sequence selected from SEQ ID NO 344 to 366).

Optionally, the second partial effector sequence described in this section may comprise one or two amino acid variations independently selected from a deletion, an insertion and a variable substitution.

### Substitution Rules:

Optionally, the variable substitution may be selected from a substitution according to the following rules:
a. glycine (G) and alanine (A) are interchangeable; valine (V), leucine (L), and isoleucine (I) are interchangeable, A and V are interchangeable;
b. tryptophan (W), phenylalanine (F), and tyrosine (Y) are interchangeable;
c. serine (S) and threonine (T) are interchangeable;
d. aspartic acid (D) and glutamic acid (E) are interchangeable
e. asparagine (N) and glutamine (Q) are interchangeable; N and S are interchangeable; N and D are interchangeable; E and Q are interchangeable;
f. methionine (M) and Q are interchangeable;
g. cysteine (C), A and S are interchangeable;
h. proline (P), G and A are interchangeable;
i. arginine (R) and lysine (K) and Q are interchangeable;
j. histidine (H) and Y are interchangeable, H and N are interchangeable;
k. L and M are interchangeable, I and M are interchangeable, V and M are interchangeable;
l. E and K are interchangeable;
m. A and C are interchangeable, A and G are interchangeable; A and T are interchangeable, A and V are interchangeable;
n. R and N are interchangeable, R and E are interchangeable, R and H are interchangeable;
o. N and Q are interchangeable, N and E are interchangeable, N and G are interchangeable, N and K are interchangeable, N and T are interchangeable;
p. D and Q are interchangeable, D and S are interchangeable;
q. Q and H are interchangeable, Q and M are interchangeable, Q and S are interchangeable;
r. E and H are interchangeable, E and S are interchangeable;
s. G and S are interchangeable;
t. F and I are interchangeable, F and L are interchangeable, F and M are interchangeable;
u. K and S are interchangeable;
v. T and V are interchangeable;

In certain embodiments, the variable substitution is selected from a substitution according to rules a to I (and not according to m to v).

### Spontaneous Complementation

In certain embodiments of the modular peptide complex according to the first, second or third aspect of this invention, the first and the second partial effector sequences are capable of forming an active, circularly permuted, self-labeling protein *without* the need of a sensor module polypeptide. These spontaneously complementing first and second partial effector sequences only need to be expressed inside the same cell or expression system in order to build a protein complex and attach a halogen alkane moiety to the first partial effector sequence if the corresponding substrate is present.

In certain embodiments of this sub-aspect, the second partial effector sequence is selected from the group consisting of SEQ ID NO 20 and SEQ ID NO 24-39. The inventors have confirmed for these sequences that they are able to complement the cpHaloΔ (first partial effector sequence; the previously published one or the cpHaloΔ as disclosed herein) in absence of a direct link such as may be established by a sensor polypeptide. The inventors do not exclude the possibility that any of the sequences of SEQ ID NO 006 to 343 can serve this role; to date however, confirmation of this complementing ability in absence of a linking agent was only confirmed for SEQ ID NO 20 and SEQ ID NO 24-39. The second partial effector sequences disclosed in the original cpHaloΔ publication (SEQ ID NO 004 and 005) are not able to perform this task.

### Sensor Module Polypeptide:

In certain embodiments of the modular peptide complex according to the first, second or third aspect of this invention, the first partial effector sequence and the second partial effector sequence are connected through a sensor module polypeptide.

In certain embodiments, the sensor module polypeptide is a single sensor polypeptide capable of undergoing conformational change from a first conformation to a second conformation depending on the presence or concentration of an analyte compound.

In the first conformation, the first and second partial effector sequences are in close proximity (lead to the first and second partial effector sequences constituting an active entity).

In the second conformation, the first and second partial effector sequences are not in close proximity (lead to the first and second partial effector sequences constituting an inactive (non-self-labelling) entity).

If the first and the second partial effector sequence are not capable of forming an active, circularly permuted, self-labelling protein in absence of an agent that induces their spatial proximity, they must be brought in close proximity in order to form an active protein. The sensor module polypeptide is coupled to the first and second partial effector sequence, and the sensor module polypeptide - when sensing a certain stimulus - will bring the first partial effector sequence and the second partial effector sequence close enough together that they can interact and form an active, circularly permuted, self-labelling protein.

Optionally,
- one of the first partial effector sequence and the second partial effector sequence may be attached to the C-terminus of the sensor module sequence (the single sensor polypeptide); and
- the other one of the first partial effector sequence and the second partial effector sequence may be attached to the N-terminus of the sensor module sequence (the single sensor polypeptide)
- or the first partial effector sequence and/or the second partial effector sequence are inserted into the single sensor polypeptide.

In certain embodiments, the sensor module polypeptide is a sensor polypeptide pair comprising a first sensor polypeptide and a second sensor polypeptide, wherein the first sensor polypeptide is covalently attached through a peptide bond to the first partial effector sequence and the second sensor polypeptide is covalently attached to the second partial effector sequence. The first sensor polypeptide and the second sensor polypeptide are capable of specific molecular interaction (protein-protein binding) with optionally, the specific molecular interaction could be dependent on an analyte concentration, and the first and second sensor polypeptides are part of separate polypeptide chains.

Using the sensor module polypeptide, it is also possible to detect co-localization of two polypeptides or peptides inside a cell, or outside of a cell, for example expressed by a cell free expression system. The two (poly)peptides of interest constitute a sensor polypeptide pair, and if they co-localize, the first and second partial effector sequences are brought into close proximity and are then capable of forming an active protein which may attach a HaloTag substrate to the first partial effector sequence.

Non-limiting examples of a sensor polypeptide are
a) calmodulin. The protein calmodulin is an example for a single sensor polypeptide. Calmodulin flanked by the first partial effector sequence and the second partial effector sequence (one at the N-terminus and one at the C-terminus of calmodulin). If calmodulin senses calcium, calmodulin will perform a conformational change and as a result, the first partial effector sequence and the second partial effector sequence are brought in close proximity and can interact.
b) FKBP/FRP. The FKBP/FRP polypeptides are an example for a sensor polypeptide pair. One of the proteins FKBP and FRB is coupled to the first partial effector sequence, and the other one is coupled to the second partial effector sequence. When rapamycin is added to the expression system, FKBP and FRB form a protein complex, and the first partial effector sequence and the second partial effector sequence are brought in close proximity and can interact.
c) intensiometric sensor of glutamate (iGluSnFR). iGluSnFR is derived from the bacterial periplasmic glutamate binding protein Gltl. The second partial effector sequence is linked to the N-terminus of iGluSnFR and the first partial effector sequence to the C-terminus. When glutamate is added, iGluSnFR will perform a conformational change and as a result, the first partial effector sequence and the second partial effector sequence are brought in close proximity and can interact.

### Further entities:

A further aspect of the invention relates to a nucleic acid sequence, or a plurality of nucleic acid sequences, encoding a modular polypeptide complex according to any one of the preceding aspects. In certain embodiments, the nucleic acid sequence is integrated into the genome of a cell.

A further aspect of the invention relates to a nucleic acid expression system comprising the nucleic acid sequence or the plurality of nucleic acid sequences, according to the previous aspect, each nucleic acid sequence being under control of a promoter sequence.

In certain embodiments, the nucleic acid expression system is a plasmid.

In certain embodiments of the nucleic acid sequence or of the nucleic acid expression system, the sequence encoding the modular polypeptide complex comprises a promoter in 5' direction of each open-reading frame encoding the modular polypeptide complex. In certain embodiments of the nucleic acid sequence or of the nucleic acid expression system, each open-reading frame encoding the modular polypeptide complex is adjacent to (in 3' or 5' direction) a multiple cloning site which allows for insertion of a coding sequence of interest in-frame with the first and/or second partial effector sequence.

A further aspect of the invention relates to a cell comprising the nucleic acid expression system according to the previous aspect, particularly wherein the promoter is operable in said cell.

A further aspect of the invention relates to a non-human transgenic animal or plant comprising a nucleic acid sequence according to the previous aspect or a nucleic acid expression system according to the previous aspect.

A further aspect of the invention relates to a kit comprising a nucleic acid sequence or a nucleic acid expression system according to the aspects above, and a HaloTag substrate. In certain embodiments, the HaloTag substrate comprises a detection moiety. In certain particular embodiments, the HaloTag substrate comprises a fluorescent dye moiety as detection moiety.

In certain embodiments, the HaloTag substrate comprises a purification tag. In certain particular embodiments, the purification tag is selected from the group of a T7-tag, a Calmodulin-binding peptide, a FLAG epitope, a GST tag, an HA tag, a polyhistidine tag, a Myc tag, biotin, and a strep-tag. Likewise, a reactive group such as groups known in the context of "click" chemistry can be part of the HaloTag substrate. Non-limiting examples thereof alkynyl (particularly ethinyl) or azide moiety, a trans-cyclooctene moiety, a tetrazine moiety, an N-hydroxysuccinimide (NHS) moiety, a maleimide moiety.

A further aspect of the invention relates to a nucleic acid sequence comprising a peptide-coding sequence element which encodes a peptide sequence selected from the group of SEQ ID NO 006-110, 112-194, 196-343. The peptide-coding sequence element is flanked by a cloning site which is situated immediately at the 5' end or at the 3' end of the peptide-coding sequence element, and allows for insertion of a coding sequence of interest in-frame with the peptide-coding part. This aspect relates to a nucleic acid sequence which is capable of accepting an insertion of a protein of interest, or a peptide of interest, cloned in-frame with a second partial effector sequence (meaning a peptide which can form a circularly permutated self-labeling protein when combined with a cpHaloΔ protein). This fusion polypeptide of the protein of interest, or the peptide of interest with the second partial effector sequence can be detectable by adding a cpHaloΔ protein and a HaloTag substrate comprising a detection moiety. In this case, the first and the second partial effector sequences will form a circularly permutated self-labeling protein which will then label itself with the HaloTag substrate. This labelling can be read out (also later) via detecting the detection moiety attached to the HaloTag substrate.

In certain embodiments of this aspect, the peptide sequence encoded by the peptide-coding sequence element is selected from the group of SEQ ID NO 6-10, 12,13, 15-26, 28, 30, 31, 34, 40, 46, 48, 50, 75, 85, 98, 103, 112, 154, 156, 160, 167, 173, 175, 182, 189, 212, 214, 233 and 337-342.

In certain embodiments of this aspect, the peptide sequence encoded by the peptide-coding sequence element is selected from the group of SEQ ID NO: 6-24.

In certain embodiments of this aspect, the sequence encoded by the peptide-coding sequence element is selected from the group of SEQ ID NO: 20 and SEQ ID NO 24-39.

### Method for detection of a molecular interaction event:

A further aspect of the invention relates to a method for detection of a molecular interaction event comprising the steps:
a) providing an expression system being capable of expressing the modular polypeptide complex according to any one of the first, second and third aspect;
b) adding a halogen alkane moiety (HaloTag substrate) to the expression system under conditions leading to expression of the modular polypeptide, wherein the halogen alkane moiety is coupled to a detection moiety;
c) in a detection step, detecting the detection moiety coupled to the first partial effector sequence.

In certain embodiments, in the detection step, a molecular interaction event between the first sensor polypeptide and the second sensor polypeptide is detected.

In certain embodiments, in the detection step, an internal molecular interaction event (conformational change) of the single sensor polypeptide is detected.

In certain embodiments, in the detection step, co-expression of the first partial effector sequence and the second partial effector sequence is detected, wherein the first partial effector sequence and the second partial effector sequence are capable of interacting spontaneously. This method may be applied to quantify activity of a certain promotor that regulates expression of either the peptide (second partial effector sequence) or the cpHaloΔ (first partial effector sequence).

In certain embodiments, in the detection step, presence of a protein of interest is detected. The protein of interest may be fused to the first partial effector sequence. Alternatively, the protein of interest may be fused to the second partial effector sequence.

When presence of a protein of interest is detected using this method, the first partial effector sequence and the second partial effector sequence must be capable of interacting spontaneously.

In certain embodiments, the expression system is a prokaryotic or eukaryotic cell, and the protein of interest is an endogenous protein of that cell.

With this method, it can be determined, inter alia, how much of the protein of interest is present in a cell or an expression system, and where the protein of interest is located. This can be analyzed at a later stage, because the HaloTag substrate is coupled *covalently* to the first partial effector sequence when the first and second partial effector sequences interact.

As the interaction (of the first and the second partial effector sequence) can be analyzed at a later timepoint, the method is also useful to be performed in living cells or even in animals or plants. Firstly, the expression and/or localization of the protein of interest can be recorded, and at a later timepoint, the recorded expression and/or localization can be analyzed. This analysis can be performed via light emittance of a fluorescent dye as part of a detection moiety coupled to the HaloTag substrate.

In certain embodiments, the nucleic acid sequence encoding the second partial effector sequence is inserted at the 3' or at the 5' end of an endogenous gene encoding the protein of interest.

In certain embodiments, the nucleic acid sequence encoding the second partial effector sequence is inserted by a CRISPR/Cas9 complex.

In certain embodiments, the first partial effector sequence is expressed from and encoded by a plasmid.

In certain embodiments, the first partial effector sequence is introduced into the cell via a viral vector.

In certain embodiments, the second partial effector sequence is selected from the group consisting of SEQ ID NO 20 and SEQ ID NO 24-39. The inventors have confirmed for these sequences that they are able to complement the cpHaloΔ (first partial effector sequence; the previously published one or the cpHaloΔ as disclosed herein) in absence of a direct link such as may be established by a sensor polypeptide. The inventors do not exclude the possibility that any of the sequences of SEQ ID NO 006 to 343 can serve this role; to date however, confirmation of this complementing ability in absence of a linking agent was only confirmed for SEQ ID NO 20 and SEQ ID NO 24-39. The second partial effector sequences disclosed in the original cpHaloΔ publication (SEQ ID NO 004 and 005) are not able to perform this task.

In certain embodiments, the detection moiety comprises a purification tag.

In certain embodiments, the detection moiety comprises a fluorophore.

If the expression and/or localization (by microscopy) of an endogenous protein of interest (POI) is investigated in a living cell, this POI has to be tagged with a live cell compatible fluorescent tag on the genome (by genome engineering techniques, e.g. CRISPR-Cas9). This tag could be a fluorescent protein like GFP or a self-labeling protein like HaloTag. Genome engineering works significantly more efficient, the smaller the genomic modification is. Making it possible to just introduce a small peptide of a split system which can serve as a fluorescent tag after complementation with the larger split part, greatly boosts throughput and chances of success in comparison to introducing large tags.

In certain embodiments, the peptide (second partial effector sequence) is inserted into the genome at the locus of the POI and the other split part (first partial effector sequence) can be expressed from a vector or from another genomic locus. When there is no sensor module polypeptide, the split parts (first and second partial effector sequence) must feature a very high affinity to each other in order for them to spontaneously complement and stay together as a stable complex. Even though the label will not be directly coupled to the peptide (second partial effector sequence) but to the cpHaloΔ part (first partial effector sequence), the complex will stay intact and hence the label will stay on the POI. An advantage of the split-HaloTag system presently claimed is that it allows to use synthetic dyes instead of GFP which are highly preferred for (super resolution) microscopy for detection of low abundance proteins.

Wherever alternatives for single separable features such as, for example, an isotype protein or an effector sequence, or a sensor sequence are laid out herein as "embodiments", it is to be understood that such alternatives may be combined freely to form discrete embodiments of the invention disclosed herein. Thus, any of the alternative embodiments for a detectable label may be combined with any of the alternative embodiments of an isotype protein and these combinations may be combined with any effector sequence, or sensor sequence mentioned herein.

The specification further encompasses the following items:

### Items:

1. A modular polypeptide complex comprising
   - a first partial effector sequence consisting of
      ∘ an N-terminal first effector sequence part characterized by SEQ ID NO 002 or by a sequence at least (≥) 90% identical (particularly ≥93%, 95%, 97% or ≥98% identical) to SEQ ID NO 002,
      ∘ a C-terminal first effector sequence part characterized by SEQ ID NO 003 or SEQ ID NO 370, or by a sequence at least (≥) 90% identical (particularly ≥93%, 95%, 97% or ≥98% identical) to SEQ ID NO 003, or to SEQ ID NO 370;
      ∘ an internal cpHalo linker consisting of 10 to 35 amino acids, particularly consisting of 12 to 20 amino acids, more particularly of ca. 15 amino acids,
         wherein the internal cpHalo linker connects the C-terminus of the N-terminal first effector sequence part to the N-terminus of the C-terminal first effector sequence part;
      ∘ wherein optionally, the first partial effector sequence is flanked on the N-terminus and/or on the C-terminus by a tetrapeptide selected from N-terminal LKPG or EKKG (SEQ ID NOs 372, 373) and C-terminal PDYE, GDVE, PDSN or PDPQ (SEQ ID NOs 374 - 377)
   - a second partial effector sequence,
      wherein
      the first and second partial effector sequences together constitute an active, circularly permuted, self-labeling protein, and are capable, when brought into close proximity of each other, to effect covalent attachment of a halogen alkane moiety, and
      the modular polypeptide complex is characterized in that the second partial effector sequence consists of a sequence selected from the group consisting of SEQ ID NO 006-343 (any of the 338 variants),
      wherein optionally, the second partial effector sequence may comprise one or two amino acid variations independently selected from a deletion, an insertion and a variable substitution.
2. The modular polypeptide complex according to item 1, wherein the second partial effector sequence consists of a sequence selected from the group consisting of SEQ ID NO: 6-10, 12,13, 15-26, 28, 30, 31, 34, 40, 46, 48, 50, 75, 85, 98, 103, 112, 154, 156, 160, 167, 173, 175, 182, 189, 195, 212, 214, 233 and 337-342, wherein optionally, the second partial effector sequence may comprise one or two amino acid variations independently selected from a deletion, an insertion and a variable substitution.
3. The modular polypeptide complex according to item 1, wherein the second partial effector sequence consists of a sequence selected from the group consisting of
   - WREEVRKAFKLFRQ (SEQ ID NO: 25)
   - WREEVRKAFKLFRS (SEQ ID NO: 24)
   - WRETFQLFRT (SEQ ID NO: 26)
   - WREMFRLFRTGRVQ (SEQ ID NO: 27)
   - WREMFQAFRT (SEQ ID NO: 28)
   - WREMFRLFRTGQRS (SEQ ID NO: 29)
   - WREMFQLFRT (SEQ ID NO: 30)
   - WREMFRLFRT (SEQ ID NO: 20)
   - SKRDWREMFRLFRT (SEQ ID NO: 31)
   - RVMSWREMFRLFRT (SEQ ID NO: 32)
   - RMWSWREMFRLFRT (SEQ ID NO: 33)
   - WKRDWREMFRLFRT (SEQ ID NO: 34)
   - RQWTWREMFRLFRT (SEQ ID NO: 35)
   - RGWTWREMFRLFRT (SEQ ID NO: 36)
   - RMWTWREMFRLFRT (SEQ ID NO: 37)
   - RQWSWREMFRLFRT (SEQ ID NO: 38)
   - RGWSWREMFRLFRT (SEQ ID NO: 39)
   wherein optionally, the second partial effector sequence may comprise one or two amino acid variations independently selected from a deletion, an insertion and a variable substitution.
4. The modular polypeptide complex according to item 1, wherein the second partial effector sequence consists of a sequence selected from the group consisting of
   - SKRDAREMFQAFRT (SEQ ID NO: 6);
   - ARLFFQLFRT (SEQ ID NO: 7);
   - YFQGARETFQAFRT (SEQ ID NO: 8);
   - WIETFKLYRE (SEQ ID NO: 9);
   - RKKEARETFQAFRT (SEQ ID NO: 10);
   - VIETFKLFRS (SEQ ID NO: 11);
   - AREMFQLFRT (SEQ ID NO: 12);
   - AYKIFQLFRT (SEQ ID NO: 13);
   - AIRMFQLFRT (SEQ ID NO: 14);
   - WGDEARETFQAFRT (SEQ ID NO: 15);
   - AREMFQAFRT (SEQ ID NO: 16);
   - WKDEVIDAFRKFRE (SEQ ID NO: 17);
   - ERETWQAFRT (SEQ ID NO: 18);
   - WREEVRKTFKLFRQ (SEQ ID NO: 19);
   - WREMFRLFRT (SEQ ID NO: 20);
   - YFQGAREMFQAFRT (SEQ ID NO: 21);
   - WKEEVIKAFKLFRD (SEQ ID NO: 22);
   - AVNMFQLFRT (SEQ ID NO: 23);
   - WREEVRKAFKLFRS (SEQ ID NO: 24);
   wherein optionally, the second partial effector sequence may comprise one or two amino acid variations independently selected from a deletion, an insertion and a variable substitution.
5. A modular polypeptide complex comprising
   - a first partial effector sequence; and
      - a second partial effector sequence consisting of a sequence selected from
         ∘ SEQ ID NO 004 and SEQ ID NO 005, or
         ∘ the group of SEQ ID NO 006-343,
            wherein optionally, the second partial effector sequence may comprise one or two amino acid variations independently selected from a deletion, an insertion and a variable substitution,
         wherein
         the first and second partial effector sequences together constitute an active, circularly permuted, self-labeling protein, and are capable, when brought into close proximity of each other, to effect covalent attachment of a halogen alkane moiety, and
   - wherein the first partial effector sequence consists of
      ∘ an N-terminal first effector sequence part characterized by SEQ ID NO 002 or by a sequence at least (≥) 90% identical (particularly ≥93%, 95%, 97% or ≥98% identical) to SEQ ID NO 002,
      ∘ a C-terminal first effector sequence part characterized by SEQ ID NO 003 or SEQ ID NO 370, or by a sequence at least (≥) 90% identical (particularly ≥93%, 95%, 97% or ≥98% identical) to SEQ ID NO 003;
      ∘ a internal cpHalo linker consisting of 10 to 35 amino acids, particularly consisting of 20 to 26 amino acids, more particularly of 22 to 24 amino acids, wherein the internal cpHalo linker connects the C-terminus of the N-terminal first effector sequence part to the N-terminus of the C-terminal first effector sequence part;
   the modular polypeptide complex is characterized in that
   - the N-terminal first effector sequence part comprises at least one N-effector amino acid substitution selected from the group consisting of
      ∘ N217D;
      ∘ F205W;
      ∘ V184E;
      ∘ V197K;
      ∘ R254W;
      and/or
   - the C-terminal first effector sequence part comprises at least one C-effector amino acid substitution selected from the group consisting of
      ∘ F80T;
      ∘ N119H;
      ∘ K117R;
      ∘ R30P;
      ∘ G7D;
      ∘ E20S;
      and/or
   - the internal cpHalo linker is characterized by a sequence selected from the group consisting of
      ∘ RSDDPRKTQTIASKISRDLNGS (SEQ ID NO: 344);
      ∘ KGGTKRDADKAVRDTLLSLNGQ (SEQ ID NO: 345);
      ∘ GGAPRDEALKKIEKAKRDTGDQ (SEQ ID NO: 346);
      ∘ KSKYDRDQILKIIAELEKKTGGS (SEQ ID NO: 347);
      ∘ QSKYPPEWLEKVIRELLKRKNGR (SEQ ID NO: 348);
      ∘ KSKYDKRQIRDIADKIAKDNNHQ (SEQ ID NO: 349);
      ∘ GADDKTKIEKILEEIKRRWQGR (SEQ ID NO: 350);
      ∘ GTSDPRNQEIAKKLARDASTVP (SEQ ID NO: 351);
      ∘ NGADKEQIDRAIEKAKRDLNNQ (SEQ ID NO: 352);
      ∘ KGASDRDEAKKLADDIRKKKGDQ (SEQ ID NO: 353);
      ∘ NSNGHRDELEKILQTIRKQNNDI (SEQ ID NO: 354);
      ∘ LKDERQRDKALEIADRADKYPTS (SEQ ID NO: 355);
      ∘ KGAEDAKERLERGDIEKKKKEQP (SEQ ID NO: 356);
      ∘ KGAEDAKERLERGDLDRWSQEWR (SEQ ID NO: 357);
      ∘ KGAEDAKERLERRDLDKINSRNS (SEQ ID NO: 358);
      ∘ KGAEDAKERLEKGYIDDKASKQQ (SEQ ID NO: 359);
      ∘ KGAEDAKERLEKGELEKKWKDHP (SEQ ID NO: 360);
      ∘ KGAEDAKERLERGEMEKAVKHGS (SEQ ID NO: 361);
      ∘ KGAEDAKERLERDELTRDIKTYPY (SEQ ID NO: 362);
      ∘ KGAEDAKERLEQGMLEEIKKKYPE (SEQ ID NO: 363);
      ∘ KGAEDAKERLERDELTKIAKNLGG (SEQ ID NO: 364);
      ∘ KGAEDAKERLEKNALDKIAKSKGD (SEQ ID NO: 365);
      ∘ KGAEDAKERLEKNDETLKKAKDKP (SEQ ID NO: 366);
      wherein optionally, the internal cpHalo linker sequence may comprise one or two or three amino acid variations independently selected from a deletion, an insertion and a variable substitution.
5 A) The modular polypeptide according to Item 5, wherein the second partial effector sequence is selected from the group of SEQ ID NO 004 and SEQ ID NO 005.
5 B) The modular polypeptide according to Item 5, wherein the second partial effector sequence is selected from the group of SEQ ID NO 006-343.
5 C) The modular polypeptide according to Item 5, wherein the second partial effector sequence is selected from the group of SEQ ID NO: 6-10, 12,13, 15-26, 28, 30, 31, 34, 40, 46, 48, 50, 75, 85, 98, 103, 112, 154, 156, 160, 167, 173, 175, 182, 189, 195, 212, 214, 233 and 337-342.
5 D) The modular polypeptide according to Item 5, wherein the second partial effector sequence is selected from the group of SEQ ID NO 020 or 024-039.
5 E) The modular polypeptide according to Item 5, wherein the second partial effector sequence is selected from the group of SEQ ID NO 020 or 006-024.
6. The modular polypeptide complex according to item 5, wherein the first partial effector sequence comprises N-effector amino acid substitutions and/or C-effector amino acid substitutions and/or internal cpHalo linker sequences selected from the group consisting of:
   - E20S and V184E;
   - E20S, N119H, and V184E;
   - E20S, N119H, V184E, and V197K;
   - E20S, N119H, and V184E, and linker sequence KSKYDRDQILKIIAELEKKTGGS (SEQ ID NO: 347);
   - E20S, N119H, V184E, and V197K, and linker sequence KSKYDRDQILKIIAELEKKTGGS (SEQ ID NO: 347);
   more particularly the first partial effector sequence comprises E20S, N119H, V184E, and V197K, and linker sequence KSKYDRDQILKIIAELEKKTGGS (SEQ ID NO: 347).
7. A modular polypeptide complex comprising the first partial effector sequence as described in item 5 or 6 or 5A-5E, and the second partial effector sequence as described in item 1 to 4.
8. The modular polypeptide complex according to any one of the preceding items, wherein the variable substitution is selected from a substitution according to the following rules:
   a. glycine (G) and alanine (A) are interchangeable; valine (V), leucine (L), and isoleucine (I) are interchangeable, A and V are interchangeable;
   b. tryptophan (W), phenylalanine (F), and tyrosine (Y) are interchangeable;
   c. serine (S) and threonine (T) are interchangeable;
   d. aspartic acid (D) and glutamic acid (E) are interchangeable
   e. asparagine (N) and glutamine (Q) are interchangeable; N and S are interchangeable; N and D are interchangeable; E and Q are interchangeable;
   f. methionine (M) and Q are interchangeable;
   g. cysteine (C), A and S are interchangeable;
   h. proline (P), G and A are interchangeable;
   i. arginine (R) and lysine (K) and Q are interchangeable;
   j. histidine (H) and Y are interchangeable, H and N are interchangeable;
   k. L and M are interchangeable, I and M are interchangeable, V and M are interchangeable;
   l. E and K are interchangeable;
   m. A and C are interchangeable, A and G are interchangeable; A and T are interchangeable, A and V are interchangeable;
   n. R and N are interchangeable, R and E are interchangeable, R and H are interchangeable;
   o. N and Q are interchangeable, N and E are interchangeable, N and G are interchangeable, N and K are interchangeable, N and T are interchangeable;
   p. D and Q are interchangeable, D and S are interchangeable;
   q. Q and H are interchangeable, Q and M are interchangeable, Q and S are interchangeable;
   r. E and H are interchangeable, E and S are interchangeable;
   s. G and S are interchangeable;
   t. F and I are interchangeable, F and L are interchangeable, F and M are interchangeable;
   u. K and S are interchangeable;
   v. T and V are interchangeable;
   particularly wherein the variable substitution is selected from a substitution according to rules a to I.
9. The modular polypeptide complex according to any one of the preceding items, wherein the first partial effector sequence and the second partial effector sequence are connected through a sensor module polypeptide, wherein
   - the sensor module polypeptide is selected from
      a) a single sensor polypeptide capable of undergoing conformational change from a first conformation to a second conformation depending on the presence or concentration of an analyte compound, wherein
         > in the first conformation, the first and second partial effector sequences are in close proximity, and
         > in the second conformation, the first and second partial effector sequences are not in close proximity,
            and
         b) a sensor polypeptide pair comprising a first sensor polypeptide and a second sensor polypeptide, wherein the first sensor polypeptide is covalently attached through a peptide bond to the first partial effector sequence and the second sensor polypeptide is covalently attached to the second partial effector sequence,
            the first sensor polypeptide and the second sensor polypeptide are capable of specific molecular interaction,
            and the first and second sensor polypeptides are part of separate polypeptide chains.
10. The modular polypeptide complex according to any one of the preceding items, wherein optionally, the first partial effector sequence is flanked on the N-terminus and/or on the C-terminus by a tetrapeptide selected from N-terminal LKPG or EKKG (SEQ ID NOs 372, 373) and C-terminal PDYE, GDVE, PDSN or PDPQ (SEQ ID NOs 374 - 377).
11. A nucleic acid sequence, or a plurality of nucleic acid sequences, encoding a modular polypeptide complex according to any one of the preceding items.
12. A nucleic acid expression system comprising the nucleic acid sequence or the plurality of nucleic acid sequences, according to item 11, each nucleic acid sequence being under control of a promoter sequence.
13. A cell comprising the nucleic acid expression system according to item 12, particularly wherein the promoter is operable in said cell.
14. A non-human transgenic animal or plant comprising a nucleic acid sequence according to item 11 or a nucleic acid expression system according to item 12.
15. A kit comprising a nucleic acid sequence or a nucleic acid expression system according to item 12, and a HaloTag substrate.
16. A method for detection of a molecular interaction event comprising the steps:
   a) providing an expression system being capable of expressing the modular polypeptide complex according to any one of items 1 to 10;
   b) adding a halogen alkane moiety to the expression system under conditions leading to expression of the modular polypeptide, wherein the halogen alkane moiety is coupled to a detection moiety;
   c) in a detection step, detecting the detection moiety coupled to the first partial effector sequence.
17. The method according to item 16, wherein in the detection step,
   - a molecular interaction event between the first sensor polypeptide and the second sensor polypeptide, or
   - an internal molecular interaction event of the single sensor polypeptide is detected.
18. The method according to item 16, wherein in the detection step, co-expression of the first partial effector sequence and the second partial effector sequence is detected.
19. The method according to item 16, wherein in the detection step, presence of a protein of interest is detected, wherein the protein of interest is fused to
   a) the first partial effector sequence, or
   b) the second partial effector sequence.
20. The method according to item 19, wherein the expression system is a cell, and the protein of interest is an endogenous protein of that cell.
21. The method according to item 20, wherein the nucleic acid sequence encoding the second partial effector sequence is inserted at the 3' or at the 5' end of an endogenous gene encoding the protein of interest.
22. The method according to item 21, wherein the nucleic acid sequence encoding the second partial effector sequence is inserted by a CRISPR/Cas9 complex.
23. The method according to any one of items 20 to 22, wherein the first partial effector sequence is expressed from and encoded by a plasmid.
24. The method according to any one of items 20 to 22, wherein the first partial effector sequence is introduced into the cell via a viral vector.
25. The method according to any one of items 19 to 24, wherein the second partial effector sequence is selected from the group consisting of SEQ ID NO 20 and SEQ ID NO 24-39.
26. The method according to any one of items 16 to 25, wherein the detection moiety comprises a purification tag.
27. The method according to any one of items 16 to 26, wherein the detection moiety comprises a fluorophore.
28. The method according to any one of item 27, wherein in the detection step, a spatial localization of the detection moiety is determined.
29. A nucleic acid sequence comprising a peptide-coding part which encodes a peptide sequence selected from the group of SEQ ID NO 6-110, 112-194, 196-343, wherein the peptide-coding part comprises a cloning site which
   - is at the 5' end or at the 3' end of the peptide-coding part, and
   - allows for insertion of a coding sequence of interest in-frame with the peptide-coding part.
30. The nucleic acid sequence according to item 29, wherein the peptide sequence is selected from the group of SEQ ID NO 6-10, 12,13, 15-26, 28, 30, 31, 34, 40, 46, 48, 50, 75, 85, 98, 103, 112, 154, 156, 160, 167, 173, 175, 182, 189, 212, 214, 233 and 337-342.
31. The nucleic acid sequence according to item 29, wherein the peptide sequence is selected from the group of SEQ ID NO: 6-24.
32. The nucleic acid sequence according to item 29, wherein the sequence is selected from the group of SEQ ID NO: 20 and SEQ ID NO 24-39.

The invention is further illustrated by the following examples and figures, from which further embodiments and advantages can be drawn. These examples are meant to illustrate the invention but not to limit its scope.

### Description of the Figures

- Fig. 1: shows the split-HaloTag concept, the labelling reaction of cpHaloΔ in presence or absence of Hpep and the concept of split-HaloTag based recorders for biological activities.
- Fig. 2: shows that the newly identified Hpeps have higher activity than the original Hpep.
- Fig. 3: A) shows the identification of Hpeps with increased initial labelling speed. The sequences in this figure relate to SEQ ID NOs: 5-10, 12-13, 15-26, 28, 30-31, 34, 40, 46, 48, 50, 75, 85, 98, 103, 112, 154, 156, 160, 167, 173, 175, 182, 189, 195, 212, 214, 233, 337-342 in the appending ST26 sequence listing, and are also stated in table 1. B) shows selected Hpeps with increased initial labelling speed. The sequences in this figure relate to SEQ ID NOs: 27, 29, 32-33, 35-39, 343.
- Fig. 4: shows the relative initial labelling speed for selected Hpep, and the identification of Hpeps with EC50 values ranging from 124 nM to 3.0 mM. The sequences in this figure relate to SEQ ID NOs: 5-6, 12, 16, 20-22, 24-39, 47, 50, 341-342.
- Fig. 5: shows the FKBP/FRB/RAPA model system.
- Fig. 6: shows peptides with higher activity in the FKBP/FRB/RAPA model system. The sequences in this figure relate to SEQ ID Nos: 5-23 and 369 in the appending ST26 sequence listing, and are also stated in table 1.
- Fig. 7: shows mutations of sequences that boosted labelling speed and show an increase in melting temperature.
- Fig. 8: shows cpHaloΔ CP-linkers that increased labelling speed and melting temperature.
- Fig. 9: shows cpHaloΔ variants with increased labelling kinetics and melting temperature increase.
- Fig. 10: shows the relative initial labelling speed for the improved cpHaloΔ variant (E20S, N119H, V184E, and V197K, and linker sequence KSKYDRDQILKIIAELEKKTGGS; SEQ ID NO 347) with selected high affinity Hpeps. The sequences in this figure relate to SEQ ID NOs: 37-39.; see "EC50 values of Hpeps for the split-HaloTag labelling reaction" in Example 1 below.
- Fig. 11:: shows results of the testing of cpHaloΔ variants to increase affinity to Hpeps. The improved cpHaloΔ variant (E20S, N119H, V184E, and V197K, and linker sequence KSKYDRDQILKIIAELEKKTGGS; SEQ ID NO 347) was extended N- and/or C-terminally by 4 amino acids (the flanking sequences are SEQ ID 372 to 377) using sequences identified by a yeast display screen.
- Fig. 12:: shows results of the testing of certain high affinity split-HaloTag variants with the non-covalent HaloTag substrate T5-CPY. The cpHaloΔ versions are based on the improved version (E20S, N119H, V184E, and V197K, and linker sequence KSKYDRDQILKIIAELEKKTGGS; SEQ ID NO 347) + extensions (SEQ ID NO 372, 373, 374 and 375) as indicated in the figure. Peptides correspond to SEQ ID NO 31 and 34.

### Examples

### Example 1: Improved Hpep

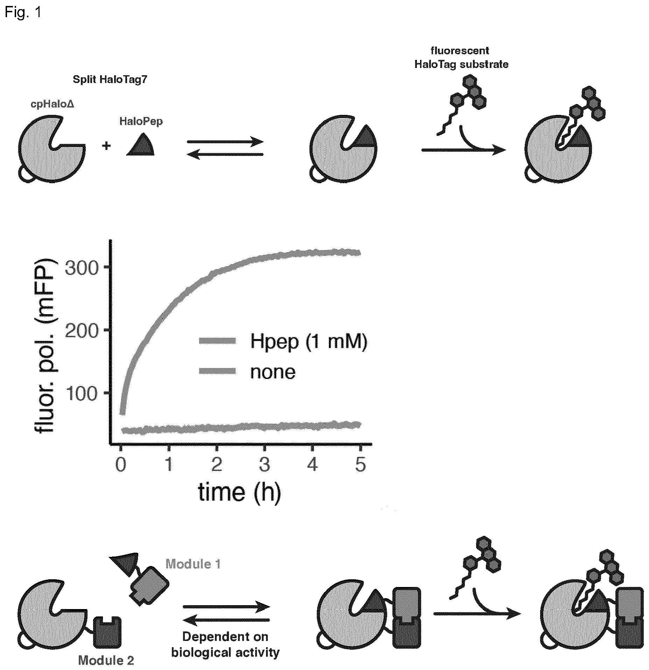

We recently developed a split system of the self labeling protein HaloTag to create recorders for transient cellular events (Fig. 1) which is described in Patent US20220275350A1 (also PCT/EP2020/060785 Hiblot et al.). We circular permutated HaloTag and found new termini at positions 154/156 or 141/145 which preserve the overall fold and labeling activity of the protein. The residues 142-155 were excised to create cpHaloΔ, which retains the overall fold of HaloTag but substantially abolished its activity. However, the activity could be restored by reversible binding of the peptide Hpep1 (residues 145-154, SEQ ID NO: 5).

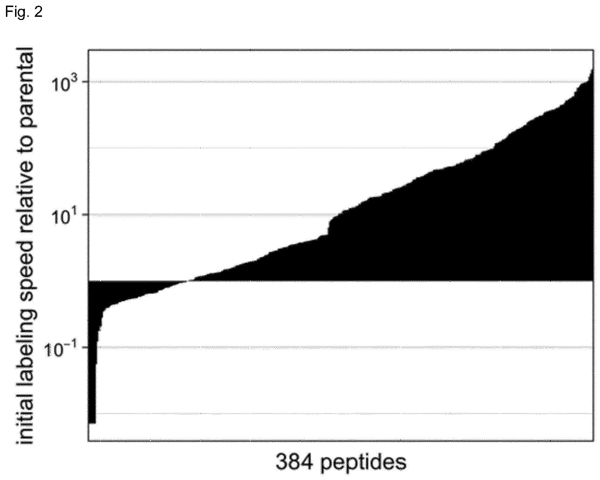

We next tested whether this split-HaloTag system can detect the rapamycin-dependent interaction of FKBP and FRB. If the split-HaloTag fragments were fused to the C-termini of FKBP and FRB, labeling was highly dependent on the presence of rapamycin. When fused to the N-termini of FKBP and FRB which are more distant (47 Å between the N-termini versus 14 Å between the C-termini), no labeling was observed. We hypothesized that this was a result of the low affinity of Hpep1 for cpHaloΔ (K_{D} = 4.6 mM), which does not enable complementation of the split fragments across longer distances. Hence, we computationally designed peptides with higher affinities for cpHaloΔ by setting up a custom RosettaScripts protocol. 40 000 peptide sequences were generated and ranked by Rosetta total score and binding free energy of the peptides. 384 selected peptide sequences were synthesized and tested for their potency to activate purified cpHaloΔ protein. 80 % of those peptides enable faster labeling of cpHaloΔ than Hpep1 (Fig. 2).

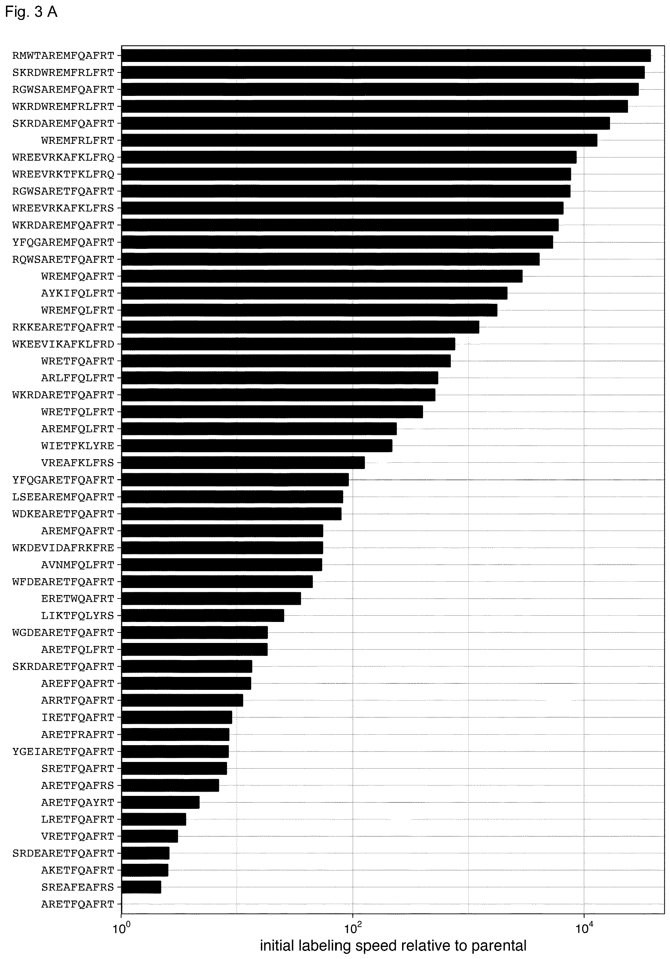

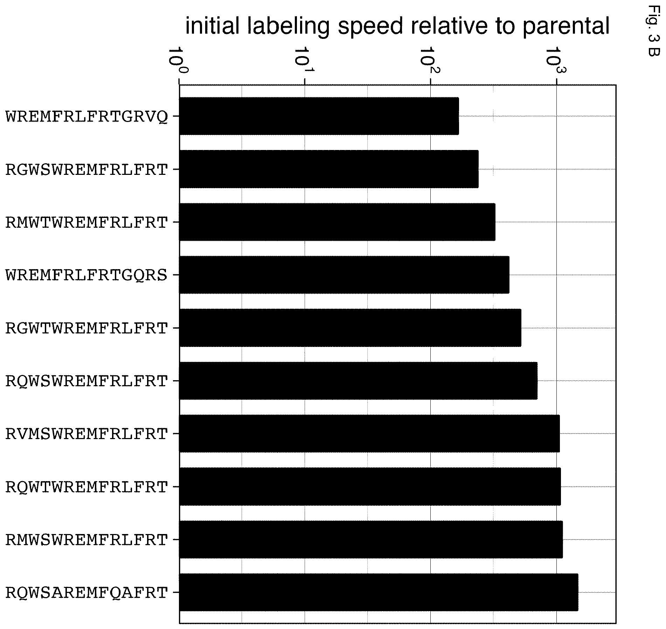

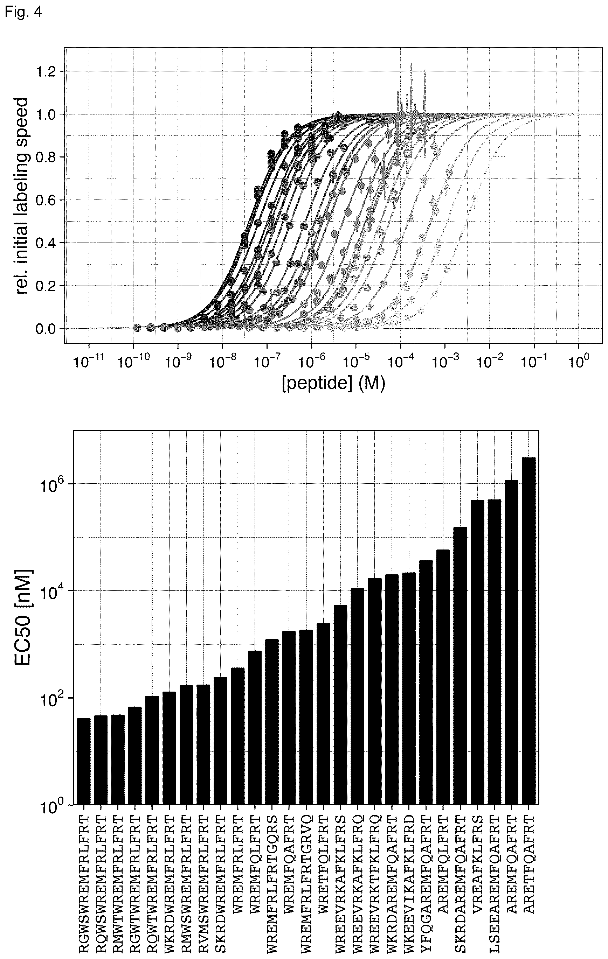

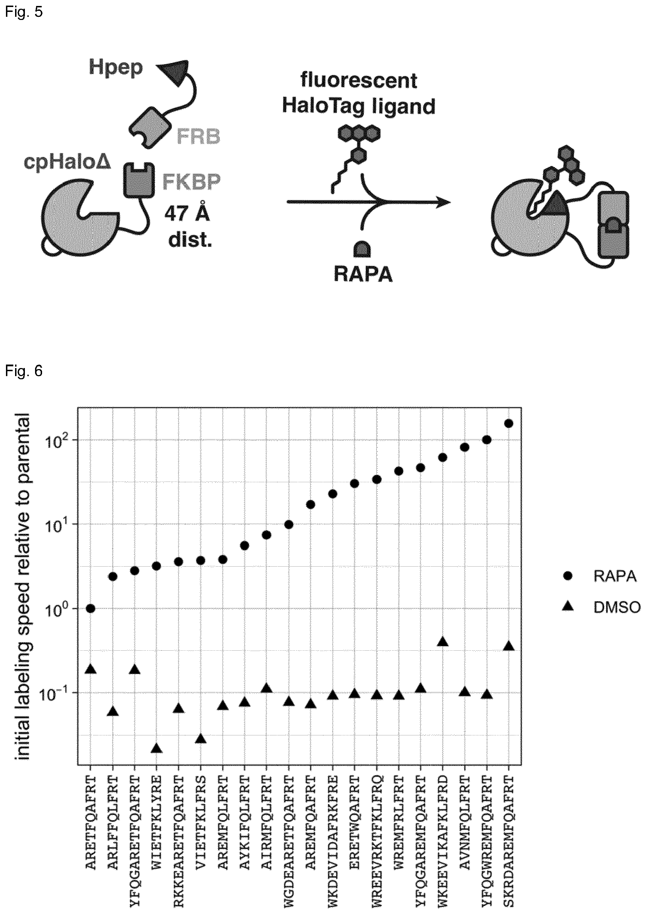

Promising peptides were purified and additional peptides bearing combinations of beneficial features (mutations or extensions) were synthesized and purified. Other candidate peptides identified by yeast surface display and phage display were also synthesized and purified. 60 of these peptides showed faster labeling of cpHaloΔ than Hpep1, with some reaching up to 30 000-fold increased initial labeling speed (Fig. 3). In order to estimate the affinity of these improved peptides for cpHaloΔ we determined EC₅₀ values of the labeling reaction for a selection of 26 peptides. Many of these peptides showed efficient labeling even at low µM or nM concentrations, conditions at which Hpep1 has no detectable activity, and featured EC₅₀ values ranging from 124 nM to 3.0 mM (Fig. 4). We then fused a selection of improved Hpeps and cpHaloΔ to the distant N-termini of FKBP and FRB and observed efficient labeling of the complex in the presence of rapamycin for several variants (>100-fold increase compared to Hpep1) while no labeling was detectable in the absence of rapamycin (Fig. 5, 6). Hence, access to different Hpeps with varying affinities considerably facilitates applications of the split-HaloTag system and largely increases its scope by making it compatible with protein-protein interactions that do not feature adjacent termini.

To also test the usability of split-HaloTag in living mammalian cells, we expressed membrane-localized FKBP-cpHaloΔ and cytosolic Hpep1-FRB in cultured HeLa cells. Membrane localized labeling of FKBP-cpHaloΔ with CPY-CA was highly conditional on the addition of rapamycin and CPY labelled cells could be identified by either fluorescence microscopy or flow cytometry analysis.

### Example 2: Improved cpHaloΔ

The labeling speed of split-HaloTag, even when using optimized Hpeps in high concentrations, is still significantly slower than that of HaloTag. To investigate this discrepancy, we measured the melting temperature (Tₘ) of the cpHaloΔ protein and found that it suffers from a greatly decreased thermal stability (Tₘ: 30.4 °C) compared to HaloTag (Tₘ: 62.0 °C). This indicates that at physiological temperature of 37 °C a large portion of the protein might not be in its active fold. To combat this issue, we aimed to stabilize cpHaloΔ via computational protein design.

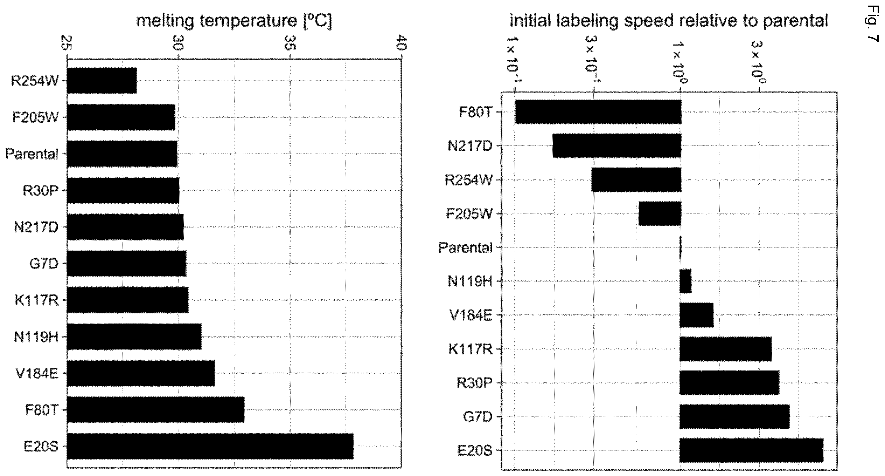

We used the PROSS server (https://pross.weizmann.ac.il/step/pross-terms/) to predict stabilizing mutations within cpHaloΔ and selected 10 mutation candidates, which were tested as single point mutants. 7 mutants had a higher melting temperature and 6 out of these featured an increased labeling speed in the presence of Hpep variant SKRDAREMFQAFRT (SEQ ID NO: 6) (Fig. 7).

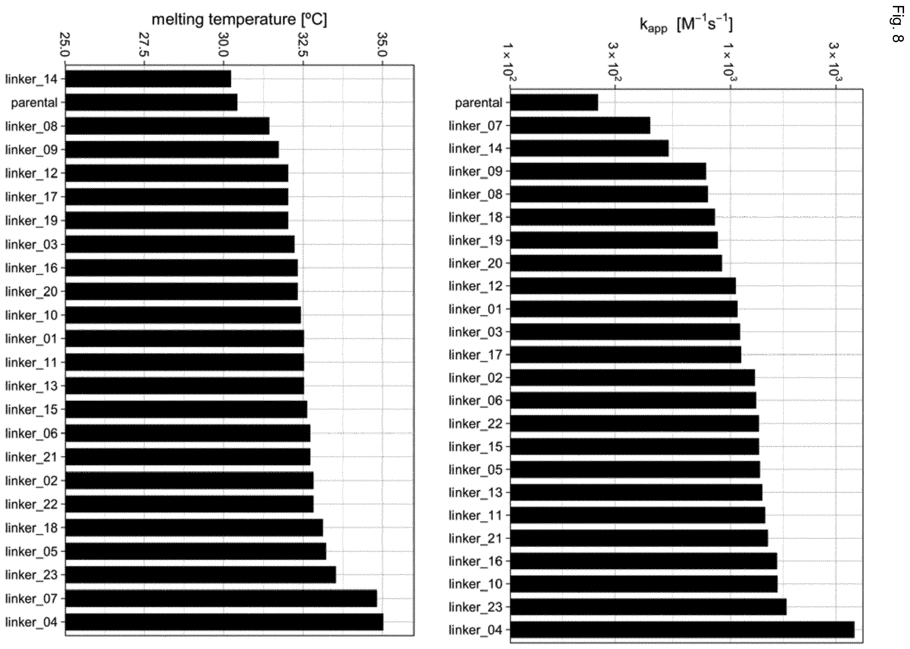

Next, we decided to re-design the CP linker, which connects the original N- and C-termini of HaloTag in cpHaloΔ using the Rosetta software suite (https://www.rosettacommons.org). We hypothesized that replacing the flexible (GGTGGSGGTGGSGGS, SEQ ID NO: 368) linker with a well folded helical linker that binds to the protein surface would increase stability. Characterizing 23 cpHaloΔ variants featuring different re-designed linkers in the presence of Hpep variant SKRDAREMFQAFRT revealed that all 23 linkers increased labeling speed (Fig. 8) and 22 linkers increased melting temperature of the protein (Fig. 8), with linker04 (KSKYDRDQILKIIAELEKKTGGS, SEQ ID NO 34) showing the best performance.

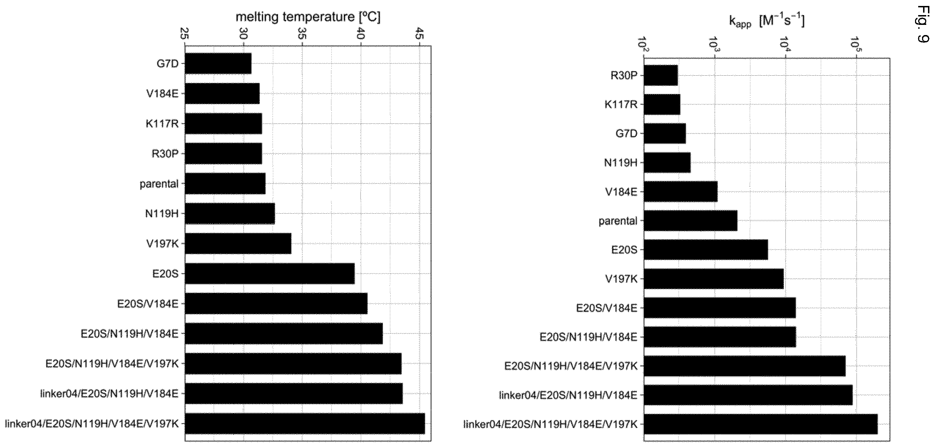

We then combined beneficial point mutations and linker04 to determine if their effects are additive to further improve stability and activity of cpHaloΔ. Indeed, several combinations showed increased Tₘ and labeling speed. The best variant (linker04/E20S/N119H/V184E/V197K) featured a 96-fold increase in labeling kinetics and a 13.6 °C increased melting temperature (Tₘ: 45.4 °C) (Fig. 9). This variant is referred to as cpHaloΔ2 in the following text.

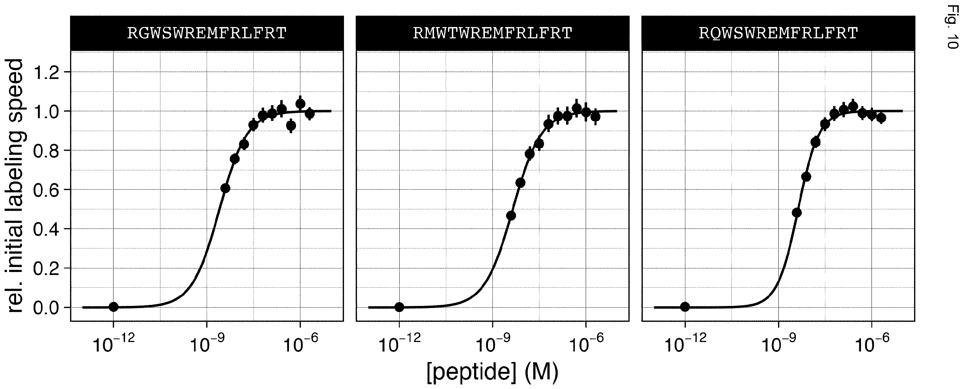

We expected that cpHaloΔ2 does not only feature higher stability and activity but would also have higher affinities towards Hpeps. To test this hypothesis, we determined EC₅₀ values for 3 high affinity Hpeps (RMWTWREMFRLFRT, SEQ ID NO: 37, RQWSWREMFRLFRT, SEQ ID NO: 38, RGWSWREMFRLFRT, SEQ ID NO: 39). On average EC₅₀ values were 12-fold lower than for the original cpHaloΔ, reaching 2.5 nM (Fig. 10). Such high affinity cpHaloΔ-Hpep pairs can be classified as spontaneously complementing split systems and open up new use cases for the split-HaloTag system.

### Example 3: Improved cpHaloΔ variants to increase affinity to Hpeps

A particularly promising application of a spontaneously complementing split-HaloTag would be labeling of endogenous proteins expressed in living cells. The endogenous protein could be tagged with Hpep by genome engineering, while cpHaloΔ2 could be expressed from a vector or another genomic site. The split-HaloTag would then spontaneously complement and the endogenous protein could be labelled with HaloTag ligands. Since it is relatively easy to introduce a short DNA sequence encoding the small Hpep to endogenous loci, compared to introducing larger tags (like full HaloTag or fluorescent proteins), this could greatly facilitate tagging of endogenous protein with bright and stable fluorescent HaloTag substrates.

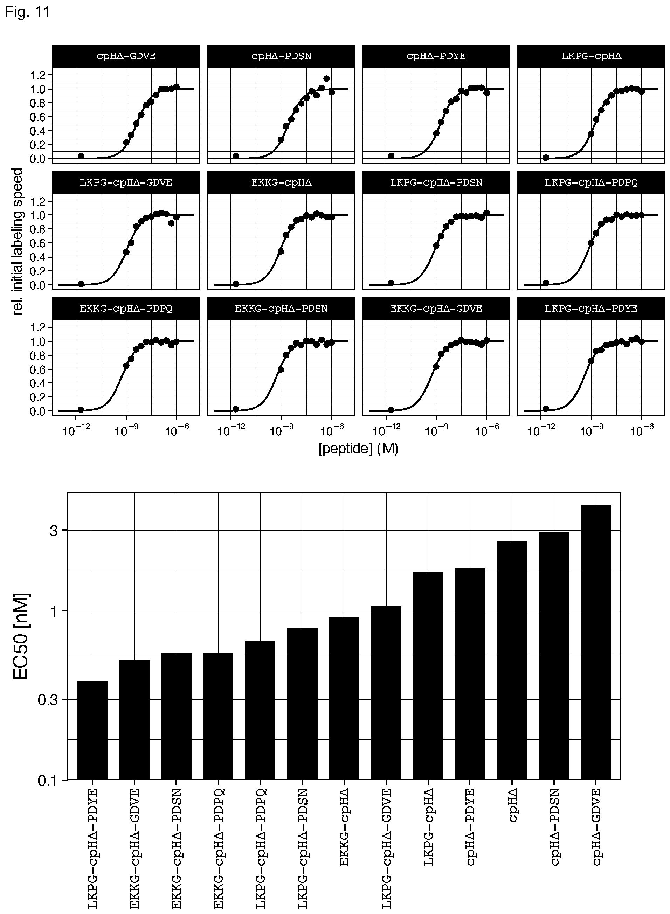

To further improve the spontaneous complementation between cpHaloΔ2 and Hpep RGWSWREMFRLFRT (SEQ ID NO 34) we extended the N- or C-terminus of cpHaloΔ2 by 4 amino acids and screened for high affinity binders via yeast surface display. Promising N- and C-terminal sequences were combined and EC₅₀ values of these extended cpHaloΔ variants with Hpep RGWSWREMFRLFRT (SEQ ID NO: 39) were determined. The best variant (LKPG-cpHaloΔ2-PDYE) (the flanking sequences are SEQ ID 372 and 374) featured a 6.6-fold lower EC₅₀ (382 pM) than cpHaloΔ2 (Fig. 11). This sub nanomolar affinity should improve the performance of split-HaloTag as a tool for tagging endogenous proteins in living cells.

### Example 4: Testing of high affinity split-HaloTag variants with non-covalent HaloTag substrates

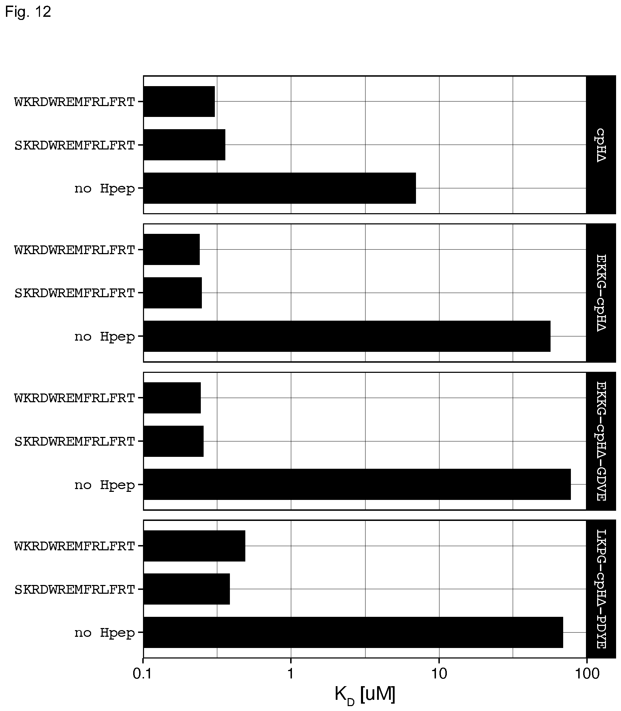

A major advantage of using split-HaloTag to label endogenous proteins over other split systems like split-GFP is that fluorescent HaloTag ligands are superior for challenging imaging technologies like super resolution microscopy (e.g. STED, PAINT, MINFLUX). This is especially the case when using exchangeable HaloTag ligands (xHTLs) which reduce photobleaching effects. Hence, we tested whether the spontaneously complementing split-HaloTag variants are compatible with xHTLs by measuring xHTL (T5-CPY) affinity in the presence or absence of Hpeps WKRDWREMFRLFRT (SEQ ID NO: 34) and SKRDWREMFRLFRT (SEQ ID NO: 31). All tested cpHaloΔ variants showed significantly stronger xHTL binding in the presence of either Hpep. The best split-HaloTag pair featured K_{D} values down to 238 nM in presence of Hpep an up to 315-fold difference in affinity between presence and absence of Hpep (Fig. 12).

### Material and Methods:

### Melting temperatures via nanoDSF

Thermostability of proteins was measured at 20 µM in activity buffer (50 mM HEPES, 50 mM NaCl, pH 7.3) on a Prometheus NT 48 nanoscale differential scanning fluorimeter (NanoTemper) over a temperature range from 20 °C to 95 °C with a heating rate of 1 °C·min⁻¹ by monitoring changes in the ratio of the fluorescence intensities at 350 nm and 330 nm. The indicated melting temperature (mean of 2 samples) corresponds to the inflection point (maximum of the first derivative).

### General procedure for fluorescence polarization assays

The concentrations of protein(s), dye and peptide used were different for the different assays and are given in the following sections. One exemplary reaction condition for determination of labelling speed is represented by the following: Mixing 100 µL of a solution containing protein at 400 nM and peptide at 10 µM with 100 µL of fluorescent HaloTag substrate (i.e. Halo-CPY) at 100 nM in a 96 well plate (black - not binding - flat bottom) in buffer (50 mM NaCl, 50 mM HEPES, pH 7.3, 0.5 mg/ml BSA). Or, mixing 20 µL of a solution containing protein at 400 nM and peptide at 10 µM with 20 µL of fluorescent HaloTag substrate (i.e. Halo-CPY) at 100 nM in a 385 well plate (black - not binding - flat bottom) in buffer (50 mM NaCl, 50 mM HEPES, pH 7.3, 0.5 mg/ml BSA). Labeling kinetics were measured by recording fluorescence polarization over time at 37 °C in a microplate reader (TECAN Spark20M). If the labeling reactions plateaued a second order reaction model was fitted to the data to obtain estimates for the apparent second order rate constants kₐₚₚ and initial slopes. If the reactions did not plateau, a linear model was fitted to obtain estimates for initial slopes.

### Initial 384 Hpep library screening

Labeling kinetics of cpHaloΔ (2 µM) with tetramethyl rhodamine HaloTag ligand (TMR-HTL, 100 nM) were measured in the presence of each peptide via fluorescence polarization readout. The peptides were ranked by initial slope of the labeling reaction in comparison to original cpHaloΔ and original Hpep (10mer).

### Characterization of purified peptides

Labeling kinetics of cpHaloΔ (500 nM) with TMR-HTL (100 nM) were measured in the presence of 10 µM of each peptide with fluorescence polarization readout. Peptides were ranked by initial slope of the labeling reaction in comparison to original cpHaloΔ and original Hpep (10mer).

### EC₅₀ values of Hpeps for the split-Halo Tag labeling reaction

Labeling kinetics of cpHaloΔ (20 nM or 500 nM) with TMR-HTL (4 nM or 100 nM) were measured with a range of Hpep concentrations (the lower cpHaloΔ and TMR-HTL concentrations were used for high affinity peptides). Initial slope of the labeling reaction was determined. The slope was plotted against Hpep concentration and a sigmoidal model was fitted to determine EC₅₀. EC₅₀: peptide concentrations at which half maximum reaction speed is observed.

### Testing new Hpeps for the capacity to label protein complexes with distant termini (FKBP/FRB)

Labeling kinetics of purified recombinant proteins (FKBP/FRB split-HaloTag fusions, 250 nM) with TMR-HTL (50 nM) were measured in presence or absence of the dimerizer rapamycin (500 nM). Initial slopes of the labeling reactions were determined and represented in comparison to corresponding constructs using the original cpHaloΔ and original Hpep (10mer).

### cpHaloΔ point mutation screening

Labeling kinetics of cpHaloΔ (100 nM) variants with TMR-HTL (20 nM) were measured in the presence of Hpep variant SKRDAREMFQAFRT (SEQ ID NO: 6) (6.25 µM). Initial slopes of the labeling reaction were determined and compared to parental protein. Melting temperatures were measured using tryptophan fluorescence (i.e. NanoDSF).

### cpHaloΔ CP-linker screening

Labeling kinetics of cpHaloΔ (100 nM) variants with TMR-HTL (20 nM) were measured in the presence of Hpep variant SKRDAREMFQAFRT (SEQ ID NO: 6) (12.5 µM). Apparent second order rate constants were determined (kₐₚₚ). Melting temperatures were measured using tryptophan fluorescence (i.e. NanoDSF).

### Characterization of cpHaloΔ variants with combinations of beneficial point mutations and CP-linkers

Labeling kinetics of cpHaloΔ (100 nM) variants with TMR-HTL (20 nM) were measured in the presence of Hpep variant SKRDAREMFQAFRT (SEQ ID NO: 6) (12.5 µM). Apparent second order rate constants were determined (kₐₚₚ). Melting temperatures were measured using tryptophan fluorescence (i.e. NanoDSF).

### EC₅₀ values of Hpep RGWSWREMFRLFRT for the split-HaloTag labeling reaction with N- and/or C-terminally extended cpHaloΔ variants

Labeling kinetics of cpHaloΔ (4 nM) with TMR-HTL (0.8 nM) were measured with a range of Hpep RGWSWREMFRLFRT (SEQ ID NO: 39) concentrations. Initial slope of the labeling reaction was determined. The slope was plotted against Hpep concentration and a sigmoidal model was fitted to determine EC₅₀. EC₅₀: peptide concentrations at which half maximum reaction speed is observed.

### Testing of high affinity split-HaloTag variants with non-covalent HaloTag substrates (xHTL)

Binding of xHTL T5-CPY (10 nM) was measured by fluorescence polarization at different concentrations (0.00128 to 100 µM) of cpHaloΔ protein alone or cpHaloΔ protein in complex with Hpeps. A sigmoidal model was fitted to data to obtain K_{D} values.

T5-CPY HaloTag substrate:

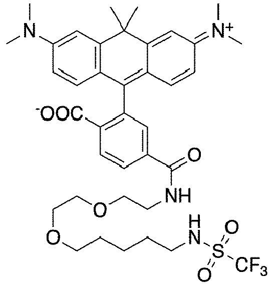

### Example 4: Sequences

**Table 1 provides the peptide sequences, also stated in the appending ST26 sequence listing.**

| **Sequence** | **SEQ ID NO:** | **Sequence** | **SEQ ID NO:** |
|---|---|---|---|
| FARETFQAFRTTDVGRKLIIDQNVFI EGTLPMGVVRPLTEVEMDHYREPF LNPVDREPLWRFPNELPIAGEPANI VALVEEYMDWLHQSPVPKLLFWG TPGVLIPPAEAARLAKSLPNCKAVDI GPGLNLLQEDNPDLIGSEIARWLST LEIGGTGGSGGTGGSGGSIGTGFPF DPHYVEVLGERMHYVDVGPRDGT PVLFLHGNPTSSYVWRNIIPHVAPT HRCIAPDLIGMGKSDKPDLGYFFD DHVRFMDAFIEALGLEEVVLVIHD WGSALGFHWAKRNPERVKGIAFM EFIRPIPTWDEW | 1 | DGEIARETFQAFRT | 201 |
| DVGRKLIIDQNVFIEGTLPMGVVRP LTEVEMDHYREPFLNPVDREPLWR FPNELPIAGEPANIVALVEEYMDW LHQSPVPKLLFWGTPGVLIPPAEAA | 2 | YGEEARETFQAFRT | 202 |
| RLAKSLPNCKAVDIGPGLNLLQEDN PDLIGSEIARWLSTLEI | | | |
| IGTGFPFDPHYVEVLGERMHYVDV GPRDGTPVLFLHGNPTSSYVWRNII PHVAPTHRCIAPDLIGMGKSDKPD LGYFFDDHVRFMDAFIEALGLEEVV LVIHDWGSALGFHWAKRNPERVK GIAFMEFIRPIPTWDEW | 3 | ESEDARETFQAFRT | 203 |
| ARETFQAFR | 4 | PKEDARETFQAFRT | 204 |
| ARETFQAFRT | 5 | FGDEARETFQAFRT | 205 |
| SKRDAREMFQAFRT | 6 | QGEEARETFQAFRT | 206 |
| ARLFFQLFRT | 7 | TGEDARETFQAFRT | 207 |
| YFQGARETFQAFRT | 8 | QAEEARETFQAFRT | 208 |
| WIETFKLYRE | 9 | FQEEARETFQAFRT | 209 |
| RKKEARETFQAFRT | 10 | TREAFEAFRS | 210 |
| VIETFKLFRS | 11 | WKEEVVKAFIKFRD | 211 |
| AREMFQLFRT | 12 | WFDEARETFQAFRT | 212 |
| AYKIFQLFRT | 13 | DGEEARETFQAFRT | 213 |
| AIRMFQLFRT | 14 | AKETFQAFRT | 214 |
| WGDEARETFQAFRT | 15 | ARETFQAFRTALKD | 215 |
| AREMFQAFRT | 16 | WEDEARETFQAFRT | 216 |
| WKDEVIDAFRKFRE | 17 | DKEEARETFQAFRT | 217 |
| ERETWQAFRT | 18 | DGDDARETFQAFRT | 218 |
| WREEVRKTFKLFRQ | 19 | FKEEARETFQAFRT | 219 |
| WREMFRLFRT | 20 | VIETFKLFRD | 220 |
| YFQGAREMFQAFRT | 21 | LIETFIQYRQ | 221 |
| WKEEVIKAFKLFRD | 22 | GRETFQAFRT | 222 |
| AVNMFQLFRT | 23 | WADEARETFQAFRT | 223 |
| WREEVRKAFKLFRS | 24 | ARETFKAFRD | 224 |
| WREEVRKAFKLFRQ | 25 | WTDEARETFQAFRT | 225 |
| WRETFQLFRT | 26 | SRETFEAFRS | 226 |
| WREMFRLFRTGRVQ | 27 | YQEEARETFQAFRT | 227 |
| WREMFQAFRT | 28 | TRETFQAFRT | 228 |
| WREMFRLFRTGQRS | 29 | AIEAFKLFRE | 229 |
| WREMFQLFRT | 30 | ARDTFQAFRT | 230 |
| SKRDWREMFRLFRT | 31 | YKEDARETFQAFRT | 231 |
| RVMSWREMFRLFRT | 32 | WREIARETFQAFRT | 232 |
| RMWSWREMFRLFRT | 33 | YGEIARETFQAFRT | 233 |
| WKRDWREMFRLFRT | 34 | FKEIARETFQAFRT | 234 |
| RQWTWREMFRLFRT | 35 | FSDEARETFQAFRT | 235 |
| RGWTWREMFRLFRT | 36 | VRDTFQAFRT | 236 |
| RMWTWREMFRLFRT | 37 | MGEDARETFQAFRT | 237 |
| RQWSWREMFRLFRT | 38 | YKEEARETFQAFRT | 238 |
| RGWSWREMFRLFRT | 39 | LIETFKKFRD | 239 |
| WKRDARETFQAFRT | 40 | GRDTFKAFRS | 240 |
| WREEVRKAFKLFRD | 41 | WKEEVIKAFILYRE | 241 |
| AIRMFQLFRE | 42 | YAEIARETFQAFRT | 242 |
| LSEEARETFQAFRT | 43 | YEEEARETFQAFRT | 243 |
| WKREARETFQAFRT | 44 | SRETFILFRQ | 244 |
| WSKEARETFQAFRT | 45 | AQETFKAFRD | 245 |
| WDKEARETFQAFRT | 46 | ARETFQAFRQ | 246 |
| WREEVRKTFKLFRS | 47 | LIKTFQLFRD | 247 |
| SKRDARETFQAFRT | 48 | DKEIARETFQAFRT | 248 |
| WKEKARETFQAFRT | 49 | LIETFRQFRE | 249 |
| VREAFKLFRS | 50 | ARDTFRAFRD | 250 |
| AREAFKAFRS | 51 | WSEMARETFQAFRT | 251 |
| DKETARETFQAFRT | 52 | ARETFQAFRTIFEH | 252 |
| DGEDARETFQAFRT | 53 | ARETFKAFRT | 253 |
| QERDARETFQAFRT | 54 | WREEARETFQAFRT | 254 |
| LRKTFKLFRS | 55 | ARETFIAFRT | 255 |
| WRETARETFQAFRT | 56 | WKDEARETFQAFRT | 256 |
| LIETFKLFRS | 57 | WGQEARETFQAFRT | 257 |
| WEETARETFQAFRT | 58 | WSDEARETFQAFRT | 258 |
| LIKTFQLFRQ | 59 | WRDEARETFQAFRT | 259 |
| WKETARETFQAFRT | 60 | ARETFQAFRD | 260 |
| AQEAFKAFRS | 61 | LIETFKLFRQ | 261 |
| IRDAFQAFRS | 62 | SSEEARETFQAFRT | 262 |
| AIKAFQLFRS | 63 | WSETARETFQAFRT | 263 |
| WDREARETFQAFRT | 64 | FKDDARETFQAFRT | 264 |
| FKQDARETFQAFRT | 65 | NGEDARETFQAFRT | 265 |
| ARDMFIKFRD | 66 | TRETFEAFRS | 266 |
| WRKEARETFQAFRT | 67 | WQEEARETFQAFRT | 267 |
| YKDDLIKTFILFRQ | 68 | SKEEARETFQAFRT | 268 |
| LGEEARETFQAFRT | 69 | AREAFEAFRS | 269 |
| YKEDLIKTFILFRQ | 70 | AIKAFQLFRE | 270 |
| WKEEVIKAFKLFRS | 71 | TRETFEAFQS | 271 |
| SRDTFQAFRS | 72 | KEEEARETFQAFRT | 272 |
| WKKEARETFQAFRT | 73 | YNEEARETFQAFRT | 273 |
| DGRDARETFQAFRT | 74 | SRDTFKAFQT | 274 |
| WRETFQAFRT | 75 | WWEEARETFQAFRT | 275 |
| WKEEVIKAFKLFRQ | 76 | AVEMFKLFRD | 276 |
| LIKAFQLFRS | 77 | MRETFQAFRT | 277 |
| SKKEARETFQAFRT | 78 | VRKTFELFRS | 278 |
| WREEVIKAFKLFRQ | 79 | SRDTFKAFQS | 279 |
| WDEEVKDAFKKFRD | 80 | YTEEARETFQAFRT | 280 |
| WKEEVIKAFRLFRE | 81 | AIEAFKLFRQ | 281 |
| LIKTFQLFRS | 82 | QKDEARETFQAFRT | 282 |
| ARKAFELFRS | 83 | KSEEARETFQAFRT | 283 |
| SRDAFKAFRS | 84 | AIETFKLFRQ | 284 |
| LIKTFQLYRS | 85 | WAEEARETFQAFRT | 285 |
| YSEEARETFQAFRT | 86 | WTKEARETFQAFRT | 286 |
| RDEDARETFQAFRT | 87 | VRDTFQAFRS | 287 |
| VRTAFKLFRS | 88 | VRKTFKLFRQ | 288 |
| SRDAFKAYRS | 89 | MGEEARETFQAFRT | 289 |
| TRDTFQAFQS | 90 | STEIARETFQAFRT | 290 |
| WEKEARETFQAFRT | 91 | SRDTFQAFQS | 291 |
| WKEEVIKAFRLYRE | 92 | VIRTFQLFRQ | 292 |
| LVETFKLFRS | 93 | SRDAFQAYRS | 293 |
| WKEDVIDAFRKFRD | 94 | LIETFKLFRT | 294 |
| LGDDARETFQAFRT | 95 | VREFFKLARD | 295 |
| MKEDARETFQAFRT | 96 | WAKEARETFQAFRT | 296 |
| VVKTFQLFRS | 97 | NRDTFQAFRS | 297 |
| ARETFQAFRS | 98 | VRETFKLFRS | 298 |
| FGKEARETFQAFRT | 99 | IRETFEAFRS | 299 |
| WKELARETFQAFRT | 100 | YKEELIKTFRLFMQ | 300 |
| WKEEVIKAFIKFRE | 101 | AIKTFILFRE | 301 |
| VREAFKLFRQ | 102 | AREAFQAFRT | 302 |
| SREAFEAFRS | 103 | KDEEARETFQAFRT | 303 |
| WKEDARETFQAFRT | 104 | LIEAFRQFRS | 304 |
| VIKTFQLFRS | 105 | VIRAFQLFRQ | 305 |
| LAEDARETFQAFRT | 106 | WGKEARETFQAFRT | 306 |
| LIETFRLFRQ | 107 | VRTAFKLFRQ | 307 |
| HKEEARETFQAFRT | 108 | SRDTFKAFRS | 308 |
| WGRDARETFQAFRT | 109 | AIETFKLFRE | 309 |
| GRETFQAFRS | 110 | VVETFKLFRS | 310 |
| ARETFQKFRT | 111 | ARDTFILFRE | 311 |
| IRETFQAFRT | 112 | VRDTFILFRS | 312 |
| QKKEARETFQAFRT | 113 | YKEDLIDTFRKFRQ | 313 |
| WKEEVIKAFILYRS | 114 | VIKAFQLFRS | 314 |
| WYEEARETFQAFRT | 115 | ARETFQAFRTAHQK | 315 |
| VIKAFQLFRQ | 116 | SSEDARETFQAFRT | 316 |
| FGREARETFQAFRT | 117 | IREAFEAFRS | 317 |
| WKEEVIKAFRLYRD | 118 | IREAFEAYRS | 318 |
| TRDTFQAFRS | 119 | SKEDARETFQAFRT | 319 |
| YKDDARETFQAFRT | 120 | ARDTFKAFRS | 320 |
| AIKTFQLFRS | 121 | AQETFKAFRS | 321 |
| ARETFQAYRS | 122 | FWEEARETFQAFRT | 322 |
| SRDTFRAFRS | 123 | VVRTFKLFRS | 323 |
| HKDEARETFQAFRT | 124 | LAEEARETFQAFRT | 324 |
| TRETFKAFRS | 125 | VIKAFIKFRE | 325 |
| SRDTFQAFRT | 126 | EKEEARETFQAFRT | 326 |
| VIKTFQLFRQ | 127 | GRDTFQAFRS | 327 |
| SRDAFQAFRS | 128 | KKEDARETFQAFRT | 328 |
| WKEEVIKAFILYRQ | 129 | ARETFQAFRTGASS | 329 |
| QREEARETFQAFRT | 130 | LIKTFILFRD | 330 |
| AQETFKAYRS | 131 | ARKTFQAFRT | 331 |
| WDRDARETFQAFRT | 132 | QKEDARETFQAFRT | 332 |
| ARDTFRAFRS | 133 | MRDEARETFQAFRT | 333 |
| MKEEARETFQAFRT | 134 | GRDTFKAFQS | 334 |
| WHEEARETFQAFRT | 135 | AIRAFQLFRQ | 335 |
| AVETFKLFRQ | 136 | YGRDARETFQAFRT | 336 |
| DKEDARETFQAFRT | 137 | RGWSAREMFQAFRT | 337 |
| FIETFKLFRD | 138 | RGWSARETFQAFRT | 338 |
| WDEDARETFQAFRT | 139 | RMWTAREMFQAFRT | 339 |
| LKEEARETFQAFRT | 140 | RQWSARETFQAFRT | 340 |
| ARDTFQAFRS | 141 | WKRDAREMFQAFRT | 341 |
| WAQEARETFQAFRT | 142 | LSEEAREMFQAFRT | 342 |
| SGEDARETFQAFRT | 143 | RQWSAREMFQAFRT | 343 |
| TREAFKAFRA | 144 | RSDDPRKTQTIASKISRDLNGS | 344 |
| LKEDARETFQAFRT | 145 | KGGTKRDADKAVRDTLLSLNGQ | 345 |
| LGEDARETFQAFRT | 146 | GGAPRDEALKKIEKAKRDTGDQ | 346 |
| YGEELIKMFIKFRD | 147 | KSKYDRDQILKIIAELEKKTGGS | 347 |
| WSEEARETFQAFRT | 148 | QSKYPPEWLEKVIRELLKRKNGR | 348 |
| MSEEARETFQAFRT | 149 | KSKYDKRQIRDIADKIAKDNNHQ | 349 |
| FSEDARETFQAFRT | 150 | GADDKTKIEKILEEIKRRWQGR | 350 |
| SKEIARETFQAFRT | 151 | GTSDPRNQEIAKKLARDASTVP | 351 |
| AIRTFQLFRQ | 152 | NGADKEQIDRAIEKAKRDLNNQ | 352 |
| WEEEARETFQAFRT | 153 | KGASDRDEAKKLADDIRKKKGDQ | 353 |
| SRDEARETFQAFRT | 154 | NSNGHRDELEKILQTIRKQNNDI | 354 |
| TRDTFQAFRT | 155 | LKDERQRDKALEIADRADKYPTS | 355 |
| SRETFQAFRT | 156 | KGAEDAKERLERGDIEKKKKEQP | 356 |
| MREDARETFQAFRT | 157 | KGAEDAKERLERGDLDRWSQEWR | 357 |
| RKEEARETFQAFRT | 158 | KGAEDAKERLERRDLDKINSRNS | 358 |
| YKEIARETFQAFRT | 159 | KGAEDAKERLEKGYIDDKASKQQ | 359 |
| ARETFQLFRT | 160 | KGAEDAKERLEKGELEKKWKDHP | 360 |
| KDETARETFQAFRT | 161 | KGAEDAKERLERGEMEKAVKHGS | 361 |
| VIRMFQLFRE | 162 | KGAEDAKERLERDELTRDIKTYPY | 362 |
| FKEDARETFQAFRT | 163 | KGAEDAKERLEQGMLEEIKKKYPE | 363 |
| LKDIARETFQAFRT | 164 | KGAEDAKERLERDELTKIAKNLGG | 364 |
| WGEIARETFQAFRT | 165 | KGAEDAKERLEKNALDKIAKSKGD | 365 |
| WGEEARETFQAFRT | 166 | KGAEDAKERLEKNDETLKKAKDKP | 366 |
| ARRTFQAFRT | 167 | DVGRKLIIDQNVFIEGTLPMGVVRPLTE VEMDHYREPFLNPVDREPLWRFPNELPI AGEPANIVALVEEYMDWLHQSPVPKLL FWGTPGVLIPPAEAARLAKSLPNCKAVD IGPGLNLLQEDNPDLIGSEIARWLSTLEIG GTGGSGGTGGSGGSIGTGFPFDPHYVE VLGERMHYVDVGPRDGTPVLFLHGNPT SSYVWRNIIPHVAPTHRCIAPDLIGMGK SDKPDLGYFFDDHVRFMDAFIEALGLEE VVLVIHDWGSALGFHWAKRNPERVKGI AFMEFIRPIPTWDEW | 367 |
| WGEDARETFQAFRT | 168 | GGTGGSGGTGGSGGS | 368 |
| KKEEARETFQAFRT | 169 | YFQGWREMFQAFRT | 369 |
| YGEDARETFQAFRT | 170 | IGTGFPFDPHYVEVLGERMHYVDVGPR DGTPVLFLHGNPTSSYVWRNIIPHVAPT HRCIAPDLIGMGKSDKPDLGYFFDDHVR | 370 |
| | | FMDAFIEALGLEEVVLVIHAWGSALGFH WAKRNPERVKGIAFMEFIRPIPTWDEW | |
| IRDTFQAYRS | 171 | DVGRKLIIDQNVFIEGTLPMGVVRPLTE VEMDHYREPFLNPVDREPLWRFPNELPI AGEPANIVALVEEYMDWLHQSPVPKLL FWGTPGVLIPPAEAARLAKSLPNCKAVD IGPGLNLLQEDNPDLIGSEIARWLSTLEIG GTGGSGGTGGSGGSIGTGFPFDPHYVE VLGERMHYVDVGPRDGTPVLFLHGNPT SSYVWRNIIPHVAPTHRCIAPDLIGMGK SDKPDLGYFFDDHVRFMDAFIEALGLEE VVLVIHAWGSALGFHWAKRNPERVKGI AFMEFIRPIPTWDEW | 371 |
| YWEEARETFQAFRT | 172 | LKPG | 372 |
| AREFFQAFRT | 173 | EKKG | 373 |
| WKEIARETFQAFRT | 174 | PDYE | 374 |
| VRETFQAFRT | 175 | GDVE | 375 |
| WDEEARETFQAFRT | 176 | PDSN | 376 |
| LIDTFRKFRS | 177 | PDPQ | 377 |
| WSEDARETFQAFRT | 178 | | |
| FSEEARETFQAFRT | 179 | | |
| QKDVARETFQAFRT | 180 | | |
| WNEEARETFQAFRT | 181 | | |
| ARETFQAYRT | 182 | | |
| FAEDARETFQAFRT | 183 | | |
| ARETFKAFRS | 184 | | |
| EKEDARETFQAFRT | 185 | | |
| LIEAFKLFRD | 186 | | |
| STEEARETFQAFRT | 187 | | |
| WTEEARETFQAFRT | 188 | | |
| LRETFQAFRT | 189 | | |
| FTEEARETFQAFRT | 190 | | |
| WDEIARETFQAFRT | 191 | | |
| FGEEARETFQAFRT | 192 | | |
| WLEEARETFQAFRT | 193 | | |
| DAEDARETFQAFRT | 194 | | |
| ARETFRAFRT | 195 | | |
| FREEARETFQAFRT | 196 | | |
| LIETFKLFRE | 197 | | |
| WKDDARETFQAFRT | 198 | | |
| YREEARETFQAFRT | 199 | | |
| VIKAFQLFRE | 200 | | |

## Claims

1. A modular polypeptide complex comprising:
- a first partial effector sequence consisting of
∘ an N-terminal first effector sequence part **characterized by** SEQ ID NO 002 or by a sequence at least (≥) 90% identical (particularly ≥93%, 95%, 97% or ≥98% identical) to SEQ ID NO 002,
∘ a C-terminal first effector sequence part **characterized by** SEQ ID NO 003 or by a sequence at least (≥) 90% identical (particularly ≥93%, 95%, 97% or ≥98% identical) to SEQ ID NO 003,
∘ an internal cpHalo linker consisting of 10 to 35 amino acids,
particularly consisting of 12 to 20 amino acids,
more particularly of ca. 15 amino acids,
wherein the internal cpHalo linker connects the C-terminus of the N-terminal first effector sequence part to the N-terminus of the C-terminal first effector sequence part;
- a second partial effector sequence,
wherein
the first and second partial effector sequences together constitute an active, circularly permuted, self-labeling protein, and are capable, when brought into close proximity of each other, to effect covalent attachment of a halogen alkane moiety, and
the modular polypeptide complex is **characterized in that** the second partial effector sequence consists of a sequence selected from the group consisting of SEQ ID NO 006 to SEQ ID NO 343,
wherein optionally, the second partial effector sequence may comprise one or two amino acid variations independently selected from a deletion, an insertion and a variable substitution.

2. The modular polypeptide complex according to claim 1, wherein the second partial effector sequence consists of a sequence selected from the group consisting of SEQ ID NO: 6-10, 12, 13, 15-26, 28, 30, 31, 34, 40, 46, 48, 50, 75, 85, 98, 103, 112, 154, 156, 160, 167, 173, 175, 182, 189, 195, 212, 214, 233 and 337-342,
wherein optionally, the second partial effector sequence may comprise one or two amino acid variations independently selected from a deletion, an insertion and a variable substitution.

3. The modular polypeptide complex according to claim 1, wherein the second partial effector sequence consists of a sequence selected from the group consisting of
- WREEVRKAFKLFRQ (SEQ ID NO: 25)
- WREEVRKAFKLFRS (SEQ ID NO: 24)
- WRETFQLFRT (SEQ ID NO: 26)
- WREMFRLFRTGRVQ (SEQ ID NO: 27)
- WREMFQAFRT (SEQ ID NO: 28)
- WREMFRLFRTGQRS (SEQ ID NO: 29)
- WREMFQLFRT (SEQ ID NO: 30)
- WREMFRLFRT (SEQ ID NO: 20)
- SKRDWREMFRLFRT (SEQ ID NO: 31)
- RVMSWREMFRLFRT (SEQ ID NO: 32)
- RMWSWREMFRLFRT (SEQ ID NO: 33)
- WKRDWREMFRLFRT (SEQ ID NO: 34)
- RQWTWREMFRLFRT (SEQ ID NO: 35)
- RGWTWREMFRLFRT (SEQ ID NO: 36)
- RMWTWREMFRLFRT (SEQ ID NO: 37)
- RQWSWREMFRLFRT (SEQ ID NO: 38)
- RGWSWREMFRLFRT (SEQ ID NO: 39)
wherein optionally, the second partial effector sequence may comprise one or two amino acid variations independently selected from a deletion, an insertion and a variable substitution.

4. The modular polypeptide complex according to claim 1, wherein the second partial effector sequence consists of a sequence selected from the group consisting of
- SKRDAREMFQAFRT (SEQ ID NO: 6);
- ARLFFQLFRT (SEQ ID NO: 7);
- YFQGARETFQAFRT (SEQ ID NO: 8);
- WIETFKLYRE (SEQ ID NO: 9);
- RKKEARETFQAFRT (SEQ ID NO: 10);
- VIETFKLFRS (SEQ ID NO: 11);
- AREMFQLFRT (SEQ ID NO: 12);
- AYKIFQLFRT (SEQ ID NO: 13);
- AIRMFQLFRT (SEQ ID NO: 14);
- WGDEARETFQAFRT (SEQ ID NO: 15);
- AREMFQAFRT (SEQ ID NO: 16);
- WKDEVIDAFRKFRE (SEQ ID NO: 17);
- ERETWQAFRT (SEQ ID NO: 18);
- WREEVRKTFKLFRQ (SEQ ID NO: 19);
- WREMFRLFRT (SEQ ID NO: 20);
- YFQGAREMFQAFRT (SEQ ID NO: 21);
- WKEEVIKAFKLFRD (SEQ ID NO: 22);
- AVNMFQLFRT (SEQ ID NO: 23);
- WREEVRKAFKLFRS (SEQ ID NO: 24);
wherein optionally, the second partial effector sequence may comprise one or two amino acid variations independently selected from a deletion, an insertion and a variable substitution.

5. A modular polypeptide complex comprising
- a first partial effector sequence; and
- a second partial effector sequence consisting of a sequence selected from the group consisting of
∘ SEQ ID NO 004 and SEQ ID NO 005, or
∘ SEQ ID NO 6 to SEQ ID NO 343;
wherein optionally, the second partial effector sequence may comprise one or two amino acid variations independently selected from a deletion, an insertion and a variable substitution,
wherein
the first and second partial effector sequences together constitute an active, circularly permuted, self-labeling protein, and are capable, when brought into close proximity of each other, to effect covalent attachment of a halogen alkane moiety, and
- wherein the first partial effector sequence consists of
∘ an N-terminal first effector sequence part **characterized by** SEQ ID NO 002 or by a sequence at least (≥) 90% identical (particularly ≥93%, 95%, 97% or ≥98% identical) to SEQ ID NO 002,
∘ a C-terminal first effector sequence part **characterized by** SEQ ID NO 003 or by a sequence at least (≥) 90% identical (particularly ≥93%, 95%, 97% or ≥98% identical) to SEQ ID NO 003,
∘ an internal cpHalo linker consisting of 10 to 35 amino acids, particularly consisting of 20 to 26 amino acids, more particularly of 22 to 24 amino acids, wherein the internal cpHalo linker connects the C-terminus of the N-terminal first effector sequence part to the N-terminus of the C-terminal first effector sequence part;
the modular polypeptide complex is **characterized in that**
• the N-terminal first effector sequence part comprises at least one N-effector amino acid substitution selected from the group consisting of
∘ N217D;
∘ F205W;
∘ V184E;
∘ V197K;
∘ R254W;
and/or
• the C-terminal first effector sequence part comprises at least one C-effector amino acid substitution selected from the group consisting of
∘ F80T;
∘ N119H;
∘ K117R;
∘ R30P;
∘ G7D;
∘ E20S;
and/or
• the internal cpHalo linker is **characterized by** a sequence selected from the group consisting of
∘ RSDDPRKTQTIASKISRDLNGS (SEQ ID NO: 344);
∘ KGGTKRDADKAVRDTLLSLNGQ (SEQ ID NO: 345);
∘ GGAPRDEALKKIEKAKRDTGDQ (SEQ ID NO: 346);
∘ KSKYDRDQILKIIAELEKKTGGS (SEQ ID NO: 347);
∘ QSKYPPEWLEKVIRELLKRKNGR (SEQ ID NO: 348);
∘ KSKYDKRQIRDIADKIAKDNNHQ (SEQ ID NO: 349);
∘ GADDKTKIEKILEEIKRRWQGR (SEQ ID NO: 350);
∘ GTSDPRNQEIAKKLARDASTVP (SEQ ID NO: 351);
∘ NGADKEQIDRAIEKAKRDLNNQ (SEQ ID NO: 352);
∘ KGASDRDEAKKLADDIRKKKGDQ (SEQ ID NO: 353);
∘ NSNGHRDELEKILQTIRKQNNDI (SEQ ID NO: 354);
∘ LKDERQRDKALEIADRADKYPTS (SEQ ID NO: 355);
∘ KGAEDAKERLERGDIEKKKKEQP (SEQ ID NO: 356);
∘ KGAEDAKERLERGDLDRWSQEWR (SEQ ID NO: 357);
∘ KGAEDAKERLERRDLDKINSRNS (SEQ ID NO: 358);
∘ KGAEDAKERLEKGYIDDKASKQQ (SEQ ID NO: 359);
∘ KGAEDAKERLEKGELEKKWKDHP (SEQ ID NO: 360);
∘ KGAEDAKERLERGEMEKAVKHGS (SEQ ID NO: 361);
∘ KGAEDAKERLERDELTRDIKTYPY (SEQ ID NO: 362);
∘ KGAEDAKERLEQGMLEEIKKKYPE (SEQ ID NO: 363);
∘ KGAEDAKERLERDELTKIAKNLGG (SEQ ID NO: 364);
∘ KGAEDAKERLEKNALDKIAKSKGD (SEQ ID NO: 365);
∘ KGAEDAKERLEKNDETLKKAKDKP (SEQ ID NO: 366);
wherein optionally, the internal cpHalo linker sequence may comprise one or two or three amino acid variations independently selected from a deletion, an insertion and a variable substitution.

6. The modular polypeptide complex according to claim 5, wherein the first partial effector sequence comprises N-effector amino acid substitutions and/or C-effector amino acid substitutions and/or internal cpHalo linker sequences selected from the group consisting of:
- E20S and V184E;
- E20S, N119H, and V184E;
- E20S, N119H, V184E, and V197K;
- E20S, N119H, and V184E, and linker sequence KSKYDRDQILKIIAELEKKTGGS (SEQ ID NO: 347);
- E20S, N119H, V184E, and V197K, and linker sequence KSKYDRDQILKIIAELEKKTGGS (SEQ ID NO: 347);
more particularly the first partial effector sequence comprises E20S, N119H, V184E, and V197K, and linker sequence KSKYDRDQILKIIAELEKKTGGS (SEQ ID NO: 347).

7. A modular polypeptide complex comprising the first partial effector sequence as described in claim 5 or 6, and the second partial effector sequence as described in claim 1 to 4.

8. The modular polypeptide complex according to any one of the preceding claims, wherein the variable substitution is selected from a substitution according to the following rules:
a. glycine (G) and alanine (A) are interchangeable; valine (V), leucine (L), and isoleucine (I) are interchangeable, A and V are interchangeable;
b. tryptophan (W), phenylalanine (F), and tyrosine (Y) are interchangeable;
c. serine (S) and threonine (T) are interchangeable;
d. aspartic acid (D) and glutamic acid (E) are interchangeable
e. asparagine (N) and glutamine (Q) are interchangeable; N and S are interchangeable; N and D are interchangeable; E and Q are interchangeable;
f. methionine (M) and Q are interchangeable;
g. cysteine (C), A and S are interchangeable;
h. proline (P), G and A are interchangeable;
i. arginine (R) and lysine (K) and Q are interchangeable;
j. histidine (H) and Y are interchangeable, H and N are interchangeable;
k. L and M are interchangeable, I and M are interchangeable, V and M are interchangeable;
l. E and K are interchangeable;
m. A and C are interchangeable, A and G are interchangeable; A and T are interchangeable, A and V are interchangeable;
n. R and N are interchangeable, R and E are interchangeable, R and H are interchangeable;
o. N and Q are interchangeable, N and E are interchangeable, N and G are interchangeable, N and K are interchangeable, N and T are interchangeable;
p. D and Q are interchangeable, D and S are interchangeable;
q. Q and H are interchangeable, Q and M are interchangeable, Q and S are interchangeable;
r. E and H are interchangeable, E and S are interchangeable;
s. G and S are interchangeable;
t. F and I are interchangeable, F and L are interchangeable, F and M are interchangeable;
u. K and S are interchangeable;
v. T and V are interchangeable;
particularly wherein the variable substitution is selected from a substitution according to rules a to I.

9. The modular polypeptide complex according to any one of the preceding claims, wherein the first partial effector sequence and the second partial effector sequence are connected through a sensor module polypeptide, wherein
- the sensor module polypeptide is selected from
a) a single sensor polypeptide capable of undergoing conformational change from a first conformation to a second conformation depending on the presence or concentration of an analyte compound, wherein
➢ in the first conformation, the first and second partial effector sequences are in close proximity, and
➢ in the second conformation, the first and second partial effector sequences are not in close proximity,
and
b) a sensor polypeptide pair comprising a first sensor polypeptide and a second sensor polypeptide, wherein the first sensor polypeptide is covalently attached through a peptide bond to the first partial effector sequence and the second sensor polypeptide is covalently attached to the second partial effector sequence,
the first sensor polypeptide and the second sensor polypeptide are capable of specific molecular interaction,
and the first and second sensor polypeptides are part of separate polypeptide chains.

10. A nucleic acid sequence, or a plurality of nucleic acid sequences, encoding a modular polypeptide complex according to any one of the preceding claims.

11. A nucleic acid expression system comprising the nucleic acid sequence or the plurality of nucleic acid sequences, according to claim 10, each nucleic acid sequence being under control of a promoter sequence.

12. A cell comprising the nucleic acid expression system according to claim 11, particularly wherein the promoter is operable in said cell.

13. A kit comprising a nucleic acid sequence or a nucleic acid expression system according to claim 11, and a HaloTag substrate.

14. A method for detection of a molecular interaction event comprising the steps:
a) providing an expression system being capable of expressing the modular polypeptide complex according to any one of claims 1 to 9;
b) adding a halogen alkane moiety to the expression system under conditions leading to expression of the modular polypeptide, wherein the halogen alkane moiety is coupled to a detection moiety;
c) in a detection step, detecting the detection moiety coupled to the first partial effector sequence.

15. The method according to claim 14, wherein in the detection step,
- a molecular interaction event between the first sensor polypeptide and the second sensor polypeptide is detected, or
- an internal molecular interaction event of the single sensor polypeptide is detected, or
- co-expression of the first partial effector sequence and the second partial effector sequence is detected, or
- presence of a protein of interest is detected, wherein the protein of interest is fused to the first partial effector sequence, or the second partial effector sequence.
